(19) Europäisches Patentamt
European Patent Office
Office européen des brevets

(11) Veröffentlichungsnummer : **0 455 052 B1**

(12) # EUROPÄISCHE PATENTSCHRIFT

(45) Veröffentlichungstag der Patentschrift :
**27.07.94 Patentblatt 94/30**

(51) Int. Cl.$^5$ : **C07D 413/06**, C07D 249/08, A01N 43/80

(21) Anmeldenummer : **91106178.6**

(22) Anmeldetag : **18.04.91**

(54) **5-(1,2,4-Triazol-1-ylmethyl)-Isoxazolin.**

(30) Priorität : **28.04.90 DE 4013723**

(43) Veröffentlichungstag der Anmeldung :
**06.11.91 Patentblatt 91/45**

(45) Bekanntmachung des Hinweises auf die Patenterteilung :
**27.07.94 Patentblatt 94/30**

(84) Benannte Vertragsstaaten :
**AT BE CH DE DK ES FR GB GR IT LI NL SE**

(56) Entgegenhaltungen :
**EP-A- 0 241 232**
**EP-A- 0 255 243**
**EP-A- 0 298 921**
**US-A- 4 749 793**
**US-A- 4 777 263**
**CHEMICAL ABSTRACTS, Band 101, Nr. 19, 5. November 1984, Seite 696, Spalte 2, Zusammenfassung-Nr. 171 181t, Columbus, Ohio, USA; L.I. VERESHCHAGIN et al.: "Reaction of 1,2,4-trazoles with propargyl bromide"**

(56) Entgegenhaltungen :
**CHEMICAL ABSTRACTS, Band 99, Nr. 23, 5. Dezember 1983, Seite 748, Spalte 2, Zusammenfassung-Nr. 194 892e, Columbus, Ohio, USA; A.A. STOTSKII et al.: "Synthesis and transformations of N-alkenyl-1,2,4-triazoles"**

(73) Patentinhaber : **BASF Aktiengesellschaft**
**Carl-Bosch-Strasse 38**
**D-67063 Ludwigshafen (DE)**

(72) Erfinder : **Zierke, Thomas, Dr.**
**Akazienstrasse 12**
**W-6737 Boehl-Iggelheim (DE)**
Erfinder : **Kuekenhoehner, Thomas, Dr.**
**Seidelstrasse 2**
**W-6710 Frankenthal (DE)**
Erfinder : **Frank, Jürgen, Dr.**
**Baltenweg 2**
**W-6703 Limburgerhof (DE)**
Erfinder : **Ammermann, Eberhard, Dr.**
**Sachsenstrasse 3**
**W-6700 Ludwigshafen (DE)**
Erfinder : **Lorenz, Gisela, Dr.**
**Erlenweg 13**
**W-6730 Neustadt (DE)**

**EP 0 455 052 B1**

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die vorliegende Erfindung betrifft neue 5-(1,2,4-Triazol-1-ylmethyl)-isoxazoline der allgemeinen Formel I

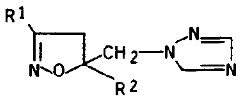

I

in der die Substituenten $R^1$ und $R^2$ die folgende Bedeutung haben:

Wasserstoff, eine $C_1$-$C_4$-Alkoxy-$C_1$-$C_4$-alkylgruppe, eine partiell oder vollständig halogenierte $C_1$-$C_6$-Alkylgruppe, eine $C_1$-$C_6$-Alkylgruppe, die einen $C_3$-$C_8$-Cycloalkyl-, Phenyl- oder Naphthylrest tragen kann, eine $C_3$-$C_8$-Cycloalkyl-, Phenyl- oder Naphthylgruppe oder eine $C_5$-$C_6$-Hetero-arylgruppe, die 3 Stickstoffatome, von denen max. 2 benachbart sein dürfen, oder bis zu 2 der folgenden Heteroatome enthält: Sauerstoff, Schwefel und/oder Stickstoff, wobei jeweils 2 Sauerstoff- und/oder Schwefelatome nicht benachbart sein dürfen, und wobei die Phenyl-, Naphthyl- und Heteroarylteile der genannten Gruppen noch jeweils einen Phenyl- oder Phenoxyrest mit bis zu 3 Halogensubstituenten oder bis zu 3 der folgenden Reste tragen können: Halogen, Cyano, Nitro, $C_1$-$C_4$-Alkyl, partiell oder vollständig halogeniertes $C_1$-$C_4$-Alkyl oder $C_1$-$C_4$-Alkoxy,

sowie die pflanzenverträglichen mineralsauren Salze und Metallkomplexe von I.

Außerdem betrifft die Erfindung ein Verfahren zur Herstellung dieser Verbindungen, ihre Verwendung als Fungizide im Pflanzenschutz und zur Regelung des Pflanzenwachstums sowie fungizide Mittel und Mittel zur Regulierung des Pflanzenwachstums, welche diese Verbindungen als wirksame Substanzen enthalten.

Des weiteren betrifft die Erfindung 2-(1,2,4-Triazol-1-ylmethyl)-alkene der Formel III′

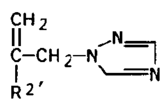

III′

in der der Substituent $R^{2'}$ die Bedeutung von $R^2$ hat, ausgenommen wasserstoff, Methyl, Phenyl und substituiertes Phenyl.

Aus der DE-A 25 51 560 sind substituierte 2-Phenyl-2-(1,2,4-Triazol-l-yl-methyl)-1,3-dioxolane und aus der EP-A 094 167 4-Phenyl-4-(1,2,4-Triazol-l-ylmethyl)-1,3-dioxolane mit fungiziden und das Wachstum der Pflanzen regulierenden Eigenschaften bekannt. Ferner werden in der genannten europäischen Druckschrift 1-(1,2,4-Triazol)-1-ylmethyl)-styrole als Zwischenprodukte für die Herstellung der dort genannten 1,3-Dioxolane beschrieben. Auch für die Styrolderivate wird eine fungizide Wirkung angegeben. Das unsubstituierte 1-(1,2,4-Triazol-1-ylmethyl)-styrol wird in der DE-A 28 33 194 beschrieben. Aus EP-A-0 241 232 sind 1-Phenyl-1-isoxazol-ethanole mit fungizider Wirkung bekannt, während aus EP-A-298 921 1,2-Benz-isoxazole und -isothiazole mit Wirkung auf dem Pharmagebiet bekannt sind. EP-A-0 255 243 betrifft 1-imidazol oder -triazol-2-phenyl-Vinylverbindungen mit fungizider Wirkung. C.A. 101, 171181 t (1984) und C.A. 99, 194892 c (1983) beschreiben Triazolmethylalkenylderivate.

Desweiteren sind aus den Druckschriften EP-A 257 351, EP-A 257 391, US-4 777 263 und US-PS 4 749 792 fungizide 3-Phenyl-3-(triazolylmethyl)- und 3-Phenyl-3-(1,3-diazolylmethyl)-isoxazolidine mit einer Methyl- bzw. Benzylgruppe am Stickstoff des (gesättigten) Isoxazolidin-Grundkörpers bekannt.

Die fungiziden und das Wachstum der Pflanzen regulierenden Eigenschaften dieser Verbindungen können jedoch, besonders bei kleinen Aufwandmengen und -konzentrationen, nur bedingt befriedigen.

Der Erfindung lag daher die Aufgabe zugrunde, neue Substanzen zu finden, die fungizide und/oder das Pflanzenwachstum regulierende Eigenschaften aufweisen.

Demgemäß wurden die eingangs definierten 5-(1,2,4-Triazol-1-ylmethyl)-isoxazoline der Formel I gefunden.

Weiterhin wurden neue Zwischenprodukte der Formel III′ zur Herstellung der 5-(1,2,4-Triazol-1-ylmethyl)-isoxazoline gefunden.

Im einzelnen haben die Substituenten $R^1$ und $R^2$ in den erfindungsgemäßen Verbindungen I die folgende Bedeutung:

- Wasserstoff;
- eine partiell oder vollständig halogenierte, verzweigte oder unverzweigte $C_1$-$C_6$-Alkylgruppe, insbesondere eine $C_1$-$C_4$-Alkylgruppe wie Chlormethyl, Dichlormethyl, Trichlormethyl, Brommethyl, 1-Chlorethyl,

Pentafluorethyl, 2-Chlor-1,1,2-trifluorethyl und 4-Chlorbut-1-yl;

- eine verzweigte oder unverzweigte $C_1$-$C_4$-Alkoxy-$C_1$-$C_4$-alkylgruppe, insbesondere eine $C_1$-$C_4$-Alkoxy-$C_1$-$C_2$-alkylgruppe wie Methoxymethyl, Ethoxymethyl, 2-Ethoxyethyl, 2-Butoxyethyl und 2-tert.-Butoxyethyl;
- eine verzweigte oder unverzweigte $C_1$-$C_6$-Alkylgruppe, insbesondere eine $C_1$-$C_4$-Alkylgruppe wie Methyl, Ethyl, Isopropyl und tert.-Butyl;
- eine Phenyl-, Naphthyl-, $C_3$-$C_8$-Cycloalkylgruppe oder eine verzweigte oder unverzweigte $C_1$-$C_6$-Alkylgruppe, insbesondere $C_1$-$C_4$-Alkylgruppe, die einen Phenyl-, Naphthyl- oder $C_3$-$C_8$-Cycloalkylrest trägt, wobei die cyclischen Reste noch jeweils einen unsubstituierten oder mit 1-3 Halogenatomen substituierten Phenyl- oder Phenoxyrest oder bis zu 2 der folgenden Substituenten tragen können: Halogen wie Fluor, Chlor und Brom; Cyano; Nitro; verzweigtes oder unverzweigtes $C_1$-$C_4$-Alkyl wie Methyl, Ethyl, Isopropyl und tert.-Butyl; verzweigtes oder unverzweigtes, partiell oder vollständig halogeniertes $C_1$-$C_4$-Alkyl wie Chlormethyl, Trichlormethyl, Trifluormethyl, Brommethyl, 1-Chlorethyl, Pentafluorethyl, 2-Chlor-1,1,2-trifluorethyl und 4-Chlor-but-1-yl oder verzweigtes oder unverzweigtes $C_1$-$C_4$-Alkoxy wie Methoxy, Ethoxy, Isopropoxy und tert.-Butoxy;
  bevorzugte Gruppen sind Cyclopropyl, 1-Methylcycloprop-1-yl, Cyclobutyl, Cyclopentyl, Cyclohexyl, 2-Methylcyclohexyl, 3-Isopropylcyclohexyl, 4-tert.-Butylcyclohexyl, 2-Isopropyl-5-methylcyclohex-1-yl, Cycloheptyl, Cyclooctyl, Cyclopropylmethyl, 1-Cyclopropylethyl, 2-Cyclopropylethyl, Cyclopentylmethyl, 1-Cyclopentylethyl, 1-Cyclohexylethyl, Phenyl, Halogenphenyl wie 2-Fluorphenyl, 2-Chlorphenyl, 2-Bromphenyl, 3-Fluorphenyl, 4-Fluorphenyl, 4-Chlorphenyl und 4-Bromphenyl, Dihalogenphenyl wie 2,3-Dichlorphenyl, 2-Chlor-3-fluorphenyl, 2,4-Dichlorphenyl, 2-Chlor-4-fluorphenyl, 2-Brom-4-fluorphenyl, 2-Brom-4-chlorphenyl, 2,6-Difluorphenyl und 2,6-Dichlorphenyl, $C_1$-$C_4$-Alkylphenyl wie 2-Methylphenyl, 4-Methylphenyl und 4-tert.-Butylphenyl, $C_1$-$C_4$-Halogenalkylphenyl wie 4-Trifluormethylphenyl, $C_1$-$C_4$-Alkoxyphenyl wie 2-Methoxyphenyl, 4-Methoxyphenyl und 4-tert.-Butoxyphenyl, sowie 2-Cyanophenyl, 4-Cyanophenyl, 2-Chlor-4-cyanophenyl, 2-Cyano-4-Chlorphenyl, 2-Nitrophenyl, 4-Nitrophenyl, 2-Chlor-4-nitrophenyl, Biphenyl, 4-Phenoxyphenyl und 4-(4′-Chlorphenoxy)-phenyl, Phenylalkyl wie Benzyl, 2-Methylbenzyl, 2-Fluorbenzyl, 2-Chlorbenzyl, 3-Fluorbenzyl, 4-Fluorbenzyl, 4-Chlorbenzyl, 2,4-Dichlorbenzyl, Phenethyl, 4-Fluorphenethyl und 4-Chlorphenethyl;
- eine $C_5$-$C_6$-Heteroarylgruppe, die 3-Stickstoffatome, von denen maximal 2 benachbart sein dürften, oder bis zu 2 der folgenden Heteroatome enthält: Sauerstoff-, Schwefel- und oder Stickstoff, wobei jeweils 2 Sauerstoff- und/oder Schwefelatome nicht benachbart sein dürften, und die noch einen Phenyl- oder Phenoxysubstituenten mit gewünschtenfalls 1 bis 3 Halogenatomen wie Fluor, Chlor und Brom, oder bis zu 2 der für die Phenyl-, Naphthyl- und Cycloalkylgruppen genannten Reste tragen kann; bevorzugte Gruppen sind Furan-2-yl, Thien-3-yl, 2-Chlorthien-3-yl, 3-Bromthien-2-yl, Pyrrol-2-yl, Pyrazol-2-yl, Isoxazol-5-yl, 3-Isopropylisoxazol-5-yl, 3-Phenylisoxazol-5-yl, Pyrid-3-yl und Pyrimid-2-yl.

$R^{2'}$ in den neuen Zwischenprodukten III′ hat die Bedeutung von $R^2$ mit der Ausnahme von Wasserstoff, Methyl, Phenyl und substituiertem Phenyl.

Besonders bevorzugte Verbindungen I und III sind den Tabellen A und B zu entnehmen.

Die 5-(1,2,4-Triazol-1-ylmethyl)-isoxazoline I sind auf verschiedene Weise erhältlich, und zwar vorzugsweise dadurch, daß man in an sich bekannter Weise (vgl. DE-A 27 54 832) Aldehydoxime IIa mit Hypochloriten in ihre Nitriloxide überführt und diese anschließend mit einem 2-(1,2,4-Triazol-1-ylmethyl)-alken III umsetzt:

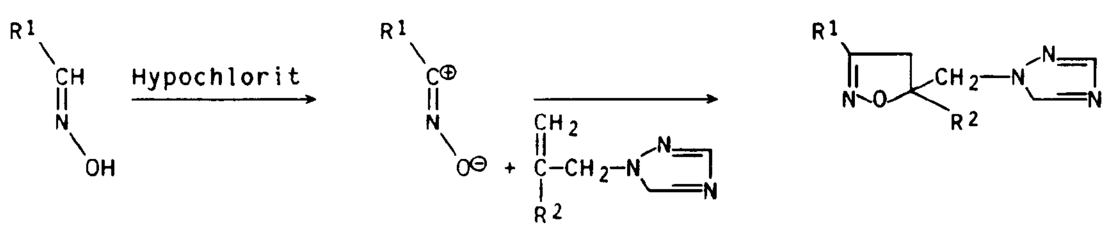


IIa IIb III I

Die Reaktion wird bevorzugt in alkalischer Lösung durchgeführt, besonders bevorzugt in einem gepufferten Zweiphasensystem aus Wasser und einem inerten organischen Lösungsmittel, insbesondere Methylenchlorid.

Vorteilhaft legt man das Oxim IIa und das Alken III vor und erzeugt das Nitriloxid IIb in situ durch Zugabe von Alkalimetall- oder Erdalkalimetallhypochlorit, insbesondere von Natrium- oder Kaliumhypochlorit.

Normalerweise setzt man die Edukte IIa und III sowie das Hypochlorit in ungefähr stöchiometrischem Verhältnis ein, jedoch kann in manchen Fällen auch ein Überschuß der einen oder anderen Komponente, etwa bis zu 30 %, vorteilhaft sein.

Im allgemeinen liegt die Reaktionstemperatur zwischen (-80)°C und dem Siedepunkt des Lösungsmittels oder Lösungsmittelgemisches, insbesondere zwischen (-20) und 40°C.

Die Umsetzung wird zweckmäßigerweise bei Normaldruck ausgeführt. Geringerer oder höherer Druck ist möglich, bringt im allgemeinen aber keine Vorteile.

Die 2-(1,2,4-Triazol-1-ylmethyl)-alkene III sind auf verschiedene Weise erhältlich, und zwar vorzugsweise nach folgenden Methoden:

a) Wittig-Reaktion von Methylen-phosphoniumyliden IV mit 1,2,4-Triazol-1-ylmethyl-ketonen V:

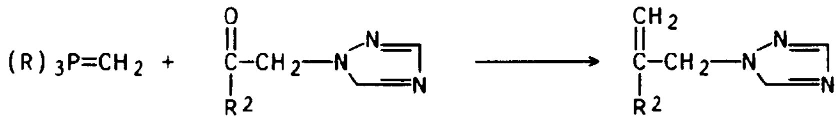

IV V III

R bedeutet einen aromatischen oder $C_6$-cycloaliphatischen Rest, insbesondere Phenyl.

Vorteilhaft überführt man ein Methylentriaryl- oder Methylentri-($C_6$-cycloalkyl)phosphoniumhalogenid, bevorzugt das Chlorid oder Bromid, mit einer starken Base in bekannter Weise in das Phosphoniumylid IV und setzt diese Verbindung in situ mit dem Keton V um.

Normalerweise arbeitet man in einem inerten Lösungsmittel, beispielsweise in aliphatischen Kohlenwasserstoffen wie Hexan und Cyclohexan, in aromatischen Kohlenwasserstoffen wie Benzol, Toluol und o-, m-, p-Xylol, in chlorierten Kohlenwasserstoffen wie Methylenchlorid, Tetrachlorkohlenstoff und Chlorbenzol, in Ethern wie Diethylether, Methyl-tert.-butylether und Tetrahydrofuran oder in Sulfoxiden wie Dimethylsulfoxid.

Als Basen eigenen sich z.B. Alkalimetallalkoholate wie Kalium-tert.-butylat, Amide wie Natriumamid und Lithiumdiisopropylamid oder Hydride wie Natrium- und Kaliumhydrid.

Zweckmäßigerweise setzt man die Phosphoniumhalogenide, Basen und Ketone V in etwa stöchiometrischem Verhältnis ein oder man verwendet einen geringen Überschuß der einen oder anderen Komponente, bis ca. 10 mol-%.

Im allgemeinen liegt die Reaktionstemperatur zwischen (-50)°C und der Rückflußtemperatur des Lösungsmittels, insbesondere zwischen 20 und 80°C.

Bezüglich des Druckes gelten die vorstehenden Angaben für die Herstellung der Isoxazoline I.

b) Umsetzung von Halogenmethyl-alkenen VI mit 1,2,4-Triazol in Gegenwart einer Base:

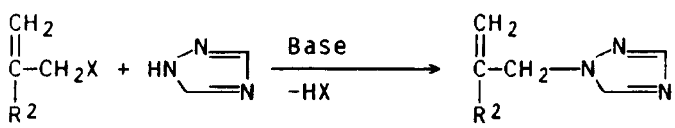


VI V III

X = Halogen, bevorzugt Chlor und Brom.

Die Synthese erfolgt normalerweise in an sich bekannter Weise [vgl. J. Org. Chem. USSR, 19, 1552 (1983)] durch Überführung des Triazols mittels Base in sein Anion und dessen anschließender Umsetzung mit einem Halogenmethyl-alken VI.

Als Basen eignen sich Alkalimetallalkoholate wie Kaliumethanolat oder Alkalimetallhydroxide wie Kaliumhydroxid.

Zweckmäßigerweise arbeitet man in einem geeigneten inerten Lösungsmittel, beispielsweise in Ethanol (insbesondere bei Verwendung von Ethanolat als Base) oder in Dimethylformamid.

Im allgemeinen werden alle Reaktionspartner in stöchiometrischen Mengen eingesetzt, jedoch kann auch ein Überschuß der einen oder anderen Komponente empfehlenswert sein. Beispielsweise kann ein Überschuß an Triazol, z.B. die 1 bis 2-fache Menge, bezogen auf die Menge an Base oder die 1 bis 5-fache, insbesondere die 1 bis 4-fache Menge, bezogen auf die Menge an Halogenmethylalken VI, verwendet werden.

Zusätzlich kann Ammoniumjodid als Katalysator zugegeben werden, z.B. in äquimolarer Menge oder in einem Überschuß bis etwa 20 %, bezogen auf die Menge an Halogenmethylalken VI.

Die Reaktionstemperatur ist nicht kritisch. Meist arbeitet man bei einer Temperatur zwischen 0 und 120°C,

insbesondere zwischen 20 und 100°C, besonders bevorzugt zwischen 20 und 30°C.

Da die Reaktion nicht druckabhängig ist, arbeitet man bevorzugt bei Normaldruck.

Die Halogenmethyl-alkene VI können nach an sich bekannten Verfahren aus den entsprechenden Hydroxymethyl-alkenen hergestellt werden. Zur Herstellung von Hydroxymethyl-alkenen sei beispielhaft auf die Literatur J. Organomet. Chem. 168, 1 (1979) verwiesen.

Die 2-(1,2,4-Triazol-1-ylmethyl)-alkene der Formel III'

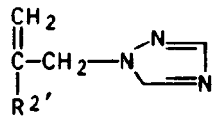

III'

wobei $R^{2'}$ die Bedeutung von $R^2$ mit Ausnahme von Wasserstoff, Methyl, Phenyl und substituiertem Phenyl hat, sind neu.

Die erfindungsgemäßen Verbindungen I enthalten mindestens ein Asymmetriezentrum. Sie wirken als Racemate, wie sie bei den meisten Herstellungsverfahren anfallen, können gewünschtenfalls aber auch nach den hierfür üblichen Methoden, z.B. durch Chromatographie an einem optisch aktiven Adsorptionsmittel, in die reinen Isomeren aufgetrennt werden.

Als Säureadditionssalze eignen sich die Salze von solchen Säuren, welche die fungizide Wirkung von I nicht beeinträchtigen, also z.B. die Hydrochloride und -bromide, Sulfate, Nitrate, Phosphate, Oxalate oder die Dodecylbenzolsulfonate.

Als Metallkomplexe kommen die Komplexe des Kupfers, Zinks, Zinns, Mangans, Eisens, Kobalts oder Nickels in Betracht. Vorzugsweise stellt man die Komplexe aus den freien Basen I und mineralsauren Salzen, beispielsweise den Chloriden oder Sulfaten, der Metalle her.

Die 5-(1,2,4-Triazol-1-ylmethyl)-isoxazoline I eignen sich als Fungizide und zur Regulierung des Pflanzenwachstums.

Die erfindungsgemäßen fungiziden und wachstumsregulierenden 5-(1,2,4-Triazol-1-ylmethyl)-isoxazoline bzw. die sie enthaltenden Mittel können beispielsweise in Form von direkt versprühbaren Lösungen, Pulvern, Suspensionen, auch hochprozentigen wäßrigen, öligen oder sonstigen Suspensionen oder Dispersionen, Emulsionen, Öldispersionen, Pasten, Stäubemitteln, Streumitteln oder Granulaten durch Versprühen, Vernebeln, Verstäuben, Verstreuen oder Gießen angewendet werden. Die Anwendungformen richten sich nach den Verwendungszwecken; sie sollten in jedem Fall möglichst die feinste Verteilung der erfindungsgemäßen Wirkstoffe gewährleisten.

Normalerweise werden die Pflanzen mit den Wirkstoffen besprüht oder bestäubt oder die Samen der Pflanzen mit den Wirkstoffen behandelt.

Die Formulierungen werden in bekannter Weise hergestellt, z.B. durch Verstrecken des Wirkstoffs mit Lösungsmitteln und/oder Trägerstoffen, gewünschtenfalls unter Verwendung von Emulgiermitteln und Dispergiermitteln, wobei im Falle von Wasser als Verdünnungsmittel auch andere organische Lösungsmittel als Hilfslösungsmittel verwendet werden können. Als Hilfsstoffe kommen dafür im wesentlichen in Betracht: Lösungsmittel wie Aromaten (z.B. Xylol), chlorierte Aromaten (z.B. Chlorbenzole), Paraffine (z.B. Erdölfraktionen), Alkohole (z.B. Methanol, Butanol), Ketone (z.B. Cyclohexanon), Amine (z.B. Ethanolamin, Dimethylformamid) und Wasser; Trägerstoffe wie natürliche Gesteinsmehle (z.B. Kaoline, Tonerden, Talkum, Kreide) und synthetische Gesteinsmehle (z.B. hochdisperse Kieselsäure, Silikate); Emulgiermittel wie nichtionogene und anionische Emulgatoren (z.B. Polyoxyethylen-Fettalkohol-Ether, Alkylsulfonate und Arylsulfonate) und Dispergiermittel wie Lignin-Sulfitablaugen und Methylcellulose.

Als oberflächenaktive Stoffe kommen die Alkali-, Erdalkali-, Ammoniumsalze von aromatischen Sulfonsäuren, z.B. Lignin-, Phenol-, Naphthalin- und Dibutylnaphthalinsulfonsäure, sowie von Fettsäuren, Alkyl- und Alkylarylsulfonaten, Alkyl-, Laurylether- und Fettalkoholsulfaten, sowie Salze sulfatierter Hexa-, Hepta- und Octadecanolen, sowie von Fettalkoholglykolether, Kondensationsprodukte von sulfoniertem Naphthalin und seiner Derivate mit Formaldehyd, Kondensationsprodukte des Naphthalins bzw. der Naphthalinsulfonsäuren mit Phenol und Formaldehyd, Polyoxyethylenoctylphenolether, ethoxyliertes Isooctyl-, Octyl- oder Nonylphenol, Alkylphenol-, Tributylphenylpolyglykolether, Alkylarylpolyetheralkohole, Isotridecylalkohol, Fettalkoholethylenoxid-Kondensate, ethoxyliertes Rizinusöl, Polyoxyethylenalkylether oder Polyoxypropylen, Laurylalkoholpolyglykoletheracetat, Sorbitester, Lignin-Sulfitablaugen oder Methylcellulose in Betracht.

Pulver-, Streu- und Stäubemittel können durch Mischen oder gemeinsames Vermahlen der wirksamen Substanzen mit einem festen Trägerstoff hergestellt werden.

Granulate, z.B. Umhüllungs-, Imprägnierungs- und Homogengranulate können durch Bindung der Wirkstoffe an feste Trägerstoffe hergestellt werden. Feste Trägerstoffe sind Mineralerden wie Silicagel, Kieselsäuren, Kieselgele, Silikate, Talkum, Kaolin, Kalkstein, Kalk, Kreide, Bolus, Löß, Ton, Dolomit, Diatomeenerde, Calcium- und Magnesiumsulfat, Magnesiumoxid, gemahlene Kunststoffe, Düngemittel, wie Ammoniumsulfat, Ammoniumphosphat, Ammoniumnitrat, Harnstoffe und pflanzliche Produkte, wie Getreidemehl, Baumrinden-, Holz- und Nußschalenmehl, Cellulosepulver oder andere feste Trägerstoffe.

Beispiele für solche Zubereitungen sind:

I. eine Lösung aus 90 Gew.-Teilen der Verbindung Nr. A.01 und 10 Gew.-Teilen N-Methyl-α-pyrrolidon, die zur Anwendung in Form kleinster Tropfen geeignet ist;

II. eine Mischung aus 20 Gew.-Teilen der Verbindung Nr. A.34, 80 Gew.Teilen Xylol, 10 Gew.-Teilen des Anlagerungsproduktes von 8 bis 10 Mol Ethylenoxid an 1 Mol Ölsäure-N-monoethanolamid, 5 Gew.Teilen Calciumsalz der Dodecylbenzolsulfonsäure, 5 Gew.-Teilen des Anlagerungsproduktes und 40 Mol Ethylenoxid an 1 Mol Ricinusöl; durch feines Verteilen der Lösung in Wasser erhält man eine Dispersion.

III. eine wäßrige Dispersion aus 20 Gew.-Teilen der Verbindung Nr. A.37, 40 Gew.-Teilen Cyclohexanon, 30 Gew.-Teilen Isobutanol, 20 Gew.-Teilen des Anlagerungsproduktes von 40 mol Ethylenoxid an 1 mol Ricinusöl;

IV. eine wäßrige Dispersion aus 20 Gew.-Teilen der Verbindung Nr. A.38, 25 Gew.-Teilen Cyclohexanol, 65 Gew.-Teilen einer Mineralölfraktion vom Siedepunkt 210 bis 280°C und 10 Gew.-Teilen des Anlagerungsproduktes von 40 mol Ethylenoxid an 1 mol Ricinusöl;

V. eine in einer Hammermühle vermahlene Mischung aus 80 Gew.-Teilen der Verbindung Nr. A.39, 3 Gew.-Teilen des Natriumsalzes der Diisobutylnaphtalin-α-sulfonsäure, 10 Gew.-Teilen des Natriumsalzes einer Ligninsulfonsäure aus einer Sulfitablauge und 7 Gew.-Teilen pulverförmigem Kieselsäuregel; durch feines Verteilen der Mischung in Wasser erhält man eine Spritzbrühe;

VI. eine innige Mischung aus 3 Gew.-Teilen der Verbindung Nr. A.53 und 97 Gew.-Teilen feinteiligem Kaolin; dieses Stäubemittel enthält 3 Gew.-% Wirkstoff;

VII. eine innige Mischung aus 30 Gew.-Teilen der Verbindung Nr. A.54, 92 Gew.-Teilen pulverförmigem Kieselsäuregel und 8 Gew.-Teilen Paraffinöl, das auf die Oberfläche dieses Kieselsäuregels gesprüht wurde; diese Aufbereitung gibt dem Wirkstoff eine gute Haftfähigkeit;

VIII. eine stabile wäßrige Dispersion aus 40 Gew.-Teilen der Verbindung Nr. A.58, 10 Gew.-Teilen des Natriumsalzes eines Phenosulfonsäure-harnstoff-formaldehyd-Kondensates, 2 Gew.-Teilen Kieselgel und 48 Gew.-Teilen Wasser, die weiter verdünnt werden kann;

IX. eine stabile ölige Dispersion aus 20 Gew.-Teilen der Verbindung Nr. A.60, 2 Gew.-Teilen des Calciumsalzes der Dodecylbenzolsulfonsäure, 8 Gew.-Teilen Fettalkohol-polyglykolether, 20 Gew.-Teilen des Natriumsalzes eines Phenolsulfonsäure-harnstoff-formaldehydKondensates und 68 Gew.-Teilen eines paraffinischen Mineralöls.

Die 5-(1,2,4-Triazol-1-ylmethyl)-isoxazoline I zeichnen sich durch eine hervorragende Wirksamkeit gegen ein breites Spektrum von pflanzenpathogenen Pilzen, insbesondere aus der Klasse der Ascomyceten und Basidiomyceten, aus. Sie sind zum Teil systemisch wirksam und können als Blatt- und Bodenfungizide eingesetzt werden.

Besondere Bedeutung haben sie für die Bekämpfung einer Vielzahl von Pilzen an verschiedenen Kulturpflanzen wie Weizen, Roggen, Gerste, Hafer, Reis, Mais, Gras, Baumwolle, Soja, Kaffee, Zuckerrohr, Wein, Obst- und Zierpflanzen und Gemüsepflanzen wie Gurken, Bohnen und Kürbisgewächsen, sowie an den Samen dieser Pflanzen.

Die Anwendung erfolgt vor oder nach der Infektion der Pflanzen oder Samen durch die Pilze.

Speziell eignen sich die Verbindungen I zur Bekämpfung folgender Pflanzenkrankheiten:

Erysiphe graminis (echter Mehltau) in Getreide,
Erysiphe cichoracearum und Sphaerotheca fuliginea an Kürbisgewächsen,
Podosphaera leucotricha an Äpfeln,
Uncinula necator an Reben,
Puccinia-Arten an Getreide,
Rhizoctonia-Arten an Baumwolle und Rasen,
Ustilago-Arten an Getreide und Zuckerrohr,
Venturia inaequalis (Schorf) an Äpfeln,
Helminthosporium-Arten an Getreide,
Septoria nodorum an Weizen,
Botrytis cinerea (Grauschimmel) an Erdbeeren, Reben,
Cercospora arachidicola an Erdnüssen,
Pseudocercosporella herpotrichoides an Weizen, Gerste,
Pyricularia oryzae an Reis,

Phytophthora infestans an Kartoffeln und Tomaten,
Fusarium- und Verticillium-Arten an verschiedenen Pflanzen,
Plasmopara viticola an Reben,
Alternaria-Arten an Gemüse und Obst.

Die fungiziden Mittel enthalten im allgemeinen zwischen 0,1 und 95, vorzugsweise zwischen 0,5 und 90 Gew.% Wirkstoff.

Die Aufwandmengen liegen je nach Art des gewünschten Effektes zwischen 0,02 und 3 kg Wirkstoff pro ha. Die neuen Verbindungen können auch im Materialschutz (Holzschutz) eingesetzt werden, z.B. gegen Paecilomyces variotii.

Bei der Saatgutbehandlung werden im allgemeinen Wirkstoffmengen von 0,001 bis 50 g, vorzugsweise 0,01 bis 10 g je Kilogramm Saatgut benötigt.

Die erfindungsgemäßen Mittel können in der Anwendungsform als Fungizide auch zusammen mit anderen Wirkstoffen vorliegen, der z.B. mit Herbiziden, Insektiziden, Wachstumsregulatoren, Fungiziden oder auch mit Düngemitteln. Beim Vermischen mit Fungiziden erhält man dabei in vielen Fällen eine Vergrößerung des fungiziden Wirkungsspektrums.

Abgesehen von ihrer Wirkung gegen Pilze können die 5-(1,2,4-Triazol-1-ylmethyl)isoxazoline auch die verschiedenen Entwicklungsstadien einer Pflanze beeinflussen und deshalb als Wachstumsregulatoren eingesetzt werden. Die Wirkungsvielfalt der Pflanzenwachstumsregulatoren ist vor allem abhängig

a) von der Pflanzenart und -sorte,
b) von dem Zeitpunkt der Applikation, bezogen auf das Entwicklungsstadium der Pflanze, und von der Jahreszeit,
c) von dem Applikationsort und -verfahren (z.B. Samenbeize, Bodenbehandlung, Blattapplikation oder Stamminjektion bei Bäumen),
d) von klimatischen Faktoren (z.B. Temperatur, Niederschlagsmenge, außerdem auch Tageslänge und Lichtintensität),
e) von der Bodenbeschaffenheit (einschließlich Düngung),
f) von der Formulierung bzw. Anwendungsform des Wirkstoffs und
g) von den angewendeten Konzentrationen der aktiven Substanz.

Aus der Reihe der verschiedenartigen Anwendungsmöglichkeiten der erfindungsgemäßen Pflanzenwachstumsregulatoren der Formel I im Pflanzenanbau, in der Landwirtschaft und im Gartenbau, werden einige nachstehend erwähnt:

A. Mit den erfindungsgemäß verwendbaren Verbindungen läßt sich das vegetative Wachstum der Pflanzen stark hemmen, was sich insbesondere in einer Reduzierung des Längenwachstums äußert. Die behandelten Pflanzen weisen demgemäß einen gedrungenen Wuchs auf; außerdem ist eine dunklere Blattfärbung zu beobachten.

Als vorteilhaft für die Praxis erweist eine verminderte Intensität des Wachstums von Gräsern an Straßenrändern, Hecken, Kanalböschungen und auf Rasenflächen wie Park-, Sport- und Obstanlagen, Zierrasen und Flugplätzen, so daß der Arbeits- und kostenaufwendige Rasenschnitt reduziert werden kann.

Von wirtschaftlichem Interesse ist auch die Erhöhung der Standfestigkeit von lageranfälligen Kulturen wie Getreide, Mais, Sonnenblumen und Soja. Die dabei verursachte Halmverkürzung und Halmverstärkung verringern oder beseitigen die Gefahr des "Lagerns" (des Umknickens) von Pflanzen unter ungünstigen Witterungsbedingungen vor der Ernte.

Wichtig ist auch die Anwendung von Wachstumsregulatoren zur Hemmung des Längenwachstums und zur zeitlichen Veränderung des Reifeverlaufs bei Baumwolle. Damit wird ein vollständig mechanisiertes Beernten dieser wichtigen Kulturpflanze ermöglicht.

Bei Obst- und anderen Bäumen lassen sich mit den Wachstumsregulatoren Schnittkosten einsparen. Außerdem kann die Alternanz von Obstbäumen durch Wachstumsregulatoren gebrochen werden.

Durch Anwendung von Wachstumsregulatoren kann auch die seitliche Verzweigung der Pflanzen vermehrt oder gehemmt werden. Daran besteht Interesse, wenn z.B. bei Tabakpflanzen die Ausbildung von Seitentrieben (Geiztrieben) zugunsten des Blattwachstums gehemmt werden soll.

Mit Wachstumsregulatoren läßt sich beispielsweise bei Winterraps auch die Frostresistenz erheblich erhöhen. Dabei werden einerseits das Längenwachstum und die Entwicklung einer zu üppigen (und dadurch besonders frostanfälligen) Blatt- bzw. Pflanzenmasse gehemmt. Andererseits werden die jungen Rapspflanzen nach der Aussaat und vor dem Einsetzen der Winterfröste trotz günstiger Wachstumsbedingungen im vegetativen Entwicklungsstadium zurückgehalten. Dadurch wird auch die Frostgefährdung solcher Pflanzen beseitigt, die zum vorzeitigen Abbau der Blühhemmung und zum Übergang in die generativen Phase neigen. Auch bei anderen Kulturen, z.B. Wintergetreide ist es vorteilhaft, wenn die Bestände durch Behandlung mit erfindungsgemäßen Verbindungen im Herbst zwar gut bestockt werden, aber nicht

zu üppig in den Winter hineingehen. Dadurch kann der erhöhten Frostempfindlichkeit und - wegen der relativ geringen Blatt- bzw. Pflanzenmasse - dem Befall mit verschiedenen Krankheiten (z.B. Pilzkrankheit) vorgebeugt werden. Die Hemmung des vegetativen Wachstums ermöglicht außerdem bei vielen Kulturpflanzen eine dichtere Bepflanzung des Bodens, so daß ein Mehrertrag bezogen auf die Bodenfläche erzielt werden kann.

B. Mit den Wachstumsregulatoren lassen sich Mehrerträge sowohl an Pflanzenteilen als auch an Pflanzeninhaltsstoffen erzielen. So ist es beispielsweise möglich, das Wachstum größerer Mengen an Knospen, Blüten, Blättern, Früchten, Samenkörnern, Wurzeln und Knollen zu induzieren, den Gehalt an Zucker in Zuckerrüben, Zuckerrohr sowie Zitrusfrüchten zu erhöhen, den Proteingehalt in Getreide oder Soja zu steigern oder Gummibäume zum vermehrten Latexfluß zu stimulieren.

Dabei können die Verbindungen der Formel I Ertragssteigerungen durch Eingriffe in den pflanzlichen Stoffwechsel bzw. durch Förderung oder Hemmung des vegetativen und/oder des generativen Wachstums verursachen.

C. Mit Pflanzenwachstumsregulatoren lassen sich schließlich sowohl eine Verkürzung bzw. Verlängerung der Entwicklungsstadien als auch eine Beschleunigung bzw. Verzögerung der Reife der geernteten Pflanzenteile vor oder nach der Ernte erreichen.

Von wirtschaftlichem Interesse ist beispielsweise die Ernteerleichterung, die durch das zeitlich konzentrierte Abfallen oder Vermindern der Haftfestigkeit am Baum bei Zitrusfrüchten, Oliven oder bei anderen Arten und Sorten von Kern-, Stein- und Schalenobst ermöglicht wird. Derselbe Mechanismus, das heißt die Förderung der Ausbildung von Trenngewebe zwischen Frucht- bzw. Blatt- und Sproßteil der Pflanze ist auch für ein gut kontrollierbares Entblättern von Nutzpflanzen wie beispielsweise Baumwolle wesentlich.

D. Mit Wachstumsregulatoren kann weiterhin der Wasserverbrauch von Pflanzen reduziert werden. Dies ist besonders wichtig für landwirtschaftliche Nutzflächen, die unter einem hohen Kostenaufwand künstlich bewässert werden müssen, z.B. in ariden oder semiariden Gebieten. Durch den Einsatz der erfindungsgemäßen Substanzen läßt sich die Intensität der Bewässerung reduzieren und damit eine kostengünstigere Bewirtschaftung durchführen. Unter dem Einfluß von Wachstumsregulatoren kommt es zu einer besseren Ausnutzung des vorhandenen Wassers, weil u.a.

- die Öffnungsweite der Stomata reduziert wird,
- eine dickere Epidermis und Cuticula ausgebildet werden,
- die Durchwurzelung des Bodens verbessert wird und
- das Mikroklima im Pflanzenbestand durch einen kompakteren Wuchs günstig beeinflußt wird.

Die erfindungsgemäß zu verwendenden Wachstumsregulatoren der Formel I können den Kulturpflanzen sowohl vom Samen her (als Saatgutbeizmittel) als auch über den Boden, d.h. durch die Wurzel sowie - besonders bevorzugt - durch Spritzung über das Blatt zugeführt werden.

Infolge der hohen Pflanzenverträglichkeit kann die Aufwandmenge stark variiert werden.

Die Aufwandmengen an Wirkstoff bei der Anwendung als Wachstumsregulator variieren je nach Bekämpfungsziel, Jahreszeit Zielpflanzen und Wachstumsstadien.

Zur Verbreiterung des Wirkungsspektrums und zur Erzielung synergistischer Effekte können die erfindungsgemäßen Verbindungen I mit zahlreichen Vertretern anderer wachstumsregulierender Wirkstoffgruppen gemischt und gemeinsam ausgebracht werden.

Außerdem kann es von Nutzen sein, die Verbindungen I allein oder in Kombination mit Herbiziden oder Fungiziden auch noch mit weiteren Pflanzenschutzmitteln gemischt gemeinsam auszubringen, beispielsweise mit Mitteln zur Bekämpfung von Schädlingen oder Bakterien. Von Interesse ist ferner die Mischbarkeit mit Mineralsalzlösungen, welche zur Behebung von Ernährungs- und Spurenelementmängeln eingesetzt werden. Es können auch nichtphytotoxische Öle und Ölkonzentrate zugesetzt werden.

Herstellungsbeispiele

Beispiel 1 (Tabellenbeispiel A.34)

3-(4-Chlorphenyl)-5-tert.-Butyl-5-(1,2,4-triazol-1-ylmethyl)-isoxazolin

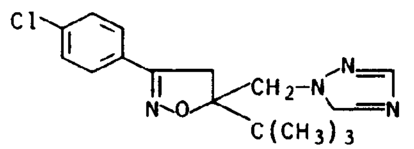

2,6 g (17 mmol) 4-Chlorbenzaldehydoxim und 3,3 g (20 mmol) 2-(1,2,4-Triazol-1-ylmethyl)-3,3-dimethylbut-1-en wurden zu einem Zweiphasensystem aus 15 ml Methylenchlorid und 15 ml eines Phosphatpuffers [enthaltend 0,3 g (1,7 mmol) $Na_2HPO_4 \times 2H_2O$ und 0,25 g (1,6 mmol) $NaH_2PO_4 \times 2H_2O$] gegeben. In diese Mischung tropfte man bei etwa 20°C 12,6 g (22 mmol) einer 13 %igen wäßrigen Natriumhypochlorid-Lösung und rührte nach beendeter Zugabe noch 3 Stunden nach. Anschließend wurde die organische Phase mit 150 ml Methylenchlorid verdünnt, nach der Phasentrennung 3 mal mit Wasser gewaschen und wie üblich auf das Produkt hin aufgearbeitet. Das Rohprodukt wurde durch Verrühren mit Diisopropylether gereinigt. Ausbeute: 53,5 %.

Vorstufe 1a (Tabellenbeispiel B.01)

2-(1, 2, 4-Triazol-1-ylmethyl)-3,3-dimethylbut-1-en

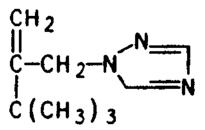

Eine Mischung aus 236,6 g (0,66 mol) Methyltriphenylphosphoniumbromid, 70,6 g (0,63 mol) Kalium-tert.-butylat und 700 g Tetrahydrofuran wurde 1 Stunde lang unter Rückfluß erhitzt. Anschließend gab man bei etwa 20°C 100 g (0,60 mol) Triazolylpinakolon zu und erhitzte weitere 2 Stunden auf 60°C. Nach dem Entfernen des Lösungsmittels wurde das Rohprodukt in Methyl-tert.-butylether aufgenommen. Die Etherphase wurde gewaschen, getrocknet und erneut eingeengt. Den Rückstand nahm man in Pentan auf und filtrierte festes Triphenylphosphinoxid ab. Danach wurde das Produkt durch Zugabe von Salpetersäure als Nitrat-Salz gefällt und isoliert. Nach Versetzen mit Natronlauge erhielt man aus dem gereinigten Salz die freie Base als langsam kristallisierendes, farbloses Öl. Ausbeute: 60,6 %.

Beispiel 2 (Tabellenbeispiel A.46)

3-(2-Chlorphenyl)-5-benzyl-5-(1,2,4-triazol-1-ylmethyl)-isoxazolin

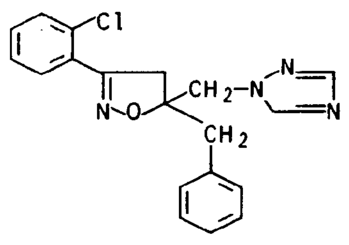

Analog Beispiel A wurden in einem 2-Phasensystem (aus 40 ml Methylenchlorid und 40 ml Phosphatpuffer) unter Einwirkung von 8,7 g (15 mmol) 13 %iger wäßriger Natriumhypochlorit-Lösung, 2,58 g (13 mmol) 2-(1,2,4-Triazol-1-ylmethyl)-3-phenyl-prop-1-en mit 2,02 g (13 mmol) 2-Chlorbenzaldehydoxim umgesetzt. Nach beendeter Zugabe des Hypochlorits rührte man die Mischung noch 16 Stunden nach und arbeitete auf das Produkt hin auf. Ausbeute 87 %.

Vorstufe 2a

2-Chlormethyl-3-phenylprop-1-en

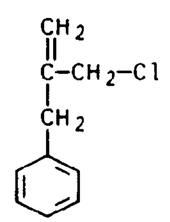

Bei 40°C wurden zu einer Mischung aus 357 g (3 mol) Thionylchlorid, 0,5 g (4 mmol) Dimethylaminopyridin und 1 1 Methylenchlorid innerhalb von 2 Stunden 148 g (1 mol) 2-Hydroxymethyl-3-phenylprop-1-en getropft. Dann erhitzte man 3 Stunden auf Rückflußtemperatur, goß die Mischung anschließend auf 1,5 1 Eiswasser und neutralisierte mit Natronlauge. Die wäßrige Phase wurde abgetrennt und 3 mal mit Methylenchlorid extrahiert, wonach die vereinigten organischen Phasen wie üblich auf das Produkt hin aufgearbeitet wurden. Ausbeute: 99 % (farbloses Öl); $^1$H-NMR (in $CDCL_3$, TMS als Standard): 3,5 ppm (s, 2H), 3,9 ppm (s, 2 H), 5,2 ppm (s, 1H), 7,1 -4 ppm (m, 1H).

Vorstufe 2b (Tabellenbeispiel B.02)

2-(1,2,4-Triazol-1-ylmethyl)-3-phenyl-prop-1-en

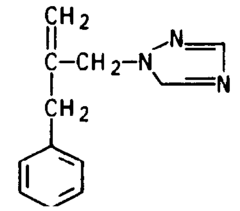

Zu einer Mischung aus 22,4 g (0,4 mol) Kaliumhydroxid-Pulver, 55,2 g (0,8 mol) 1,2,4-Triazol und 100 ml Dimethylformamid wurde bei 20°C eine Lösung von 33,3 g (0,2 mol) 2-Chlormethyl-3-phenylprop-1-en in 50 ml Dimethylformamid getropft. Nach beendeter Zugabe wurde die Reaktionsmischung noch 16 Stunden nachgerührt, dann mit 700 ml Methyl-tert.-butylether verdünnt, viermal mit Wasser gewaschen und dann wie üblich auf das Produkt hin aufgearbeitet. Ausbeute 69 % (hellgelbes Öl); $^1$H-NMR (in $CDCl_3$, TMS als Standard): 3,3 ppm (s, 2H), 4,7 ppm (s, 2H), 5,0 ppm (s, 1H), 5,1 ppm (s, 1H), 7,0-4 ppm (s, 5H), 7,95 ppm (s, 1H), 8,0 ppm (s, 1H).

Die physikalischen Daten der Endprodukte I und der neuen 2-(1,2,4-Triazol-1-ylmethyl)alkene III′ sind den Tabellen A und B zu entnehmen, in denen noch weitere Verbindungen I bzw. III′ aufgeführt sind, welche auf die gleichen Weisen hergestellt wurden oder bestellbar sind.

Tabelle A

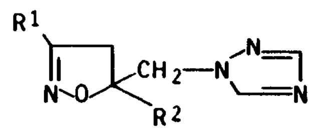

I

| Nr. | $R^1$ | $R^2$ | Physik. Daten (NMR in $CDCL_3$ [ppm]; TMS als Standard) |
|---|---|---|---|
| A.01 | Cyclopentyl | Methyl | Öl, NMR: 1.4(s, 3H), 2.7(d, 1H), 3.1(d, 1H), 4.3(s, 2H) |
| A.02 | 4-Fluorphenyl | Wasserstoff | Öl, NMR: 3.3(dd, 1H), 3.5(dd, 1H), 4.4(d, 1H) |
| A.03 | 4-Fluorphenyl | Methyl | Öl, NMR: 1.5(s, 3H), 3.1(d, 1H), 3.5(d, 1H), 4.4(s, 2H) |
| A.04 | 4-Chlorphenyl | Methyl | Öl, NMR: 1.5(s, 3H), 3.1(d, 1H), 3.5(d, 1H, 4.4(s, 2H) |
| A.05 | 2-Chlorphenyl | Methyl | Öl, NMR: 1.5(s, 3H), 3.3(d, 1H), 3.6(d, 1H), 4.4(s, 2H) |
| A.06 | 2-Chlorphenyl | Wasserstoff | Öl, NMR: 3.4(dd, 1H), 3.7(dd, 1H), 4.4(d, 2H) |
| A.07 | 2-Fluorphenyl | Wasserstoff | Öl, NMR: 3.4(ddd, 1H), 3.6(ddd, 1H), 4.4(d, 2H) |
| A.08 | 2-Fluorphenyl | Methyl | Öl, NMR: 1.5(s, 3H), 3.2(dd, 1H), 4.4(s, 2H) |
| A.09 | 3-Fluorphenyl | Methyl | Öl, NMR: 1.5(s, 3H), 3.1(d, 1H), 3.5(d, 1H), 4.4(s, 2H) |
| A.10 | 3-Chlorphenyl | Methyl | Öl, NMR: 1.5(d, 3H), 3.1(d, 1H), 3.5(d, 1H), 4.4(s, 2H) |
| A.11 | 2,4-Dichlorphenyl | Methyl | Öl, NMR: 1.5(s, 3H), 3.3(d, 1H), 3.6(d, 1H), 4.4(d, 2H) |
| A.12 | Pyrid-2-yl | Methyl | Öl, NMR: 1.5(s, 3H), 3.3(d, 1H), 3.6(d, 1H), 4.4(d, 2H) |
| A.13 | Pyrid-2-yl | Wasserstoff | Öl, NMR: 3.4(dd, 1H), 3.7(dd, 1H), 4.4(d, 2H) |
| A.14 | 4-Fluorbenzyl | Wasserstoff | Öl, NMR: 2.8(dd, 1H), 3.0(dd, 1H), 4.2-4(m, 2H) |
| A.15 | 4-Fluorbenzyl | Methyl | Öl, NMR: 1.4(s, 3H), 2.6(d, 1H), 3.0(d, 1H), 4.3(s, 2H) |

| Nr. | $R^1$ | $R^2$ | Physik. Daten (NMR in $CDCL_3$ [ppm]; TMS als Standard) |
|---|---|---|---|
| A.16 | tert.-Butyl | Methyl | Öl, NMR: 1.5(s,1H), 2.7(d,1H), 3.1(d,1H), 4.3(s,2H) |
| A.17 | tert.-Butyl | Wasserstoff | Öl, NMR: 2.9(d,1H), 3.1(d,1H), 4.2-4(m,2H) |
| A.18 | 5,5-Dimethyl-1,3-dioxan-2-yl | Wasserstoff | Öl, NMR: 3.0(dd,1H), 3.3(dd,1H), 4.3(d,2H) |
| A.19 | 5,5-Dimethyl-1,3-dioxan-2-yl | Methyl | Öl, NMR: 1.4(s,3H), 2.9(d,1H), 3.2(d,1H), 4.3(s,2H) |
| A.20 | 4-Fluorphenyl | Phenyl | Öl, NMR: 3.5(d,1H), 4.0(d,1H), 4.6(d,1H), 4.7(d,1H) |
| A.21 | 2,4-Dichlorphenyl | Phenyl | Fp: 116°C |
| A.22 | 2-Chlorphenyl | Phenyl | Fp: 157-8°C |
| A.23 | 4-Chlorphenyl | Phenyl | Fp: 98°C |
| A.24 | 2-Fluorphenyl | Phenyl | Fp: 157-8°C |
| A.25 | Phenyl | Phenyl | Fp: 133-5°C |
| A.26 | 3-Fluorphenyl | Phenyl | Fp: 156-8°C |
| A.27 | 4-Fluorphenyl | Phenyl | Fp: 103-5°C |
| A.28 | n-Propyl | Phenyl | Öl, NMR: 3.1(d,1H), 3.5(d,1H), 4.47(d,1H), 4.55(d,1H) |
| A.29 | tert.-Butyl | Phenyl | Fp: 125-7°C |
| A.30 | Cyclopropyl | Phenyl | Fp: 104-5°C |
| A.31 | Ethyl | Phenyl | Fp: 75-7°C |
| A.32 | Isopropyl | Phenyl | Fp: 87-8°C |
| A.33 | 2-Chlorphenyl | tert.-Butyl | Fp: 104°C |
| A.34 | 4-Chlorphenyl | tert.-Butyl | Fp: 157°C; NMR: 1.12(s,9H), 3.23 (d,1H), 3.43(d,1H), 4.5(d,1H), 4.6(d,1H), 7.3(d,2H), 7.38(s,2H), 7.72(s,1H), 8.25(s,1H) |
| A.35 | Phenyl | tert.-Butyl | Fp: 150°C |
| A.36 | 2-Fluorphenyl | tert.-Butyl | Fp: 115-7°C |
| A.37 | 4-Fluorphenyl | tert.-Butyl | Fp: 165°C |
| A.38 | 3-Fluorphenyl | tert.-Butyl | Fp: 162°C |
| A.39 | 4-Fluorphenyl | 4-Fluorphenyl | Fp: 98-100°C |
| A.40 | 4-Fluorphenyl | tert.-Butyl | Fp: 109°C |

| Nr. | $R^1$ | $R^2$ | Physik. Daten (NMR in $CDCL_3$ [ppm]; TMS als Standard) |
|---|---|---|---|
| A.41 | tert.-Butyl | tert.-Butyl | Fp: 138-40°C |
| A.42 | 2,4-Dichlorphenyl | tert.-Butyl | Fp: 93°C |
| A.43 | 2,6-Difluorphenyl | tert.-Butyl | Öl, NMR: 3.3(d,1H),3.4(d,1H),4.5(d,1H) |
| A.44 | 2-Methylphenyl | tert.-Butyl | Öl, NMR: 3.3(d,1H),3.5(d,1H),4.5(d,1H),4.6(d,1H) |
| A.45 | 4-Fluorphenyl | Benzyl | Öl, NMR: 3.2(d,1H),3.4(d,1H),4.4(s,2H) |
| A.46 | 2-Chlorphenyl | Benzyl | Öl, NMR: 3.1 (d,1H),3.15(d,1H),3.4(d,1H),3.5(d,1H), 4.5(s,2H),7.0-5(m,9H),7.9(s,1H),8.3(s,1H) |
| A.47 | 2,4-Dichlorphenyl | Benzyl | Öl, NMR: 3.3(d,1H),3.5(d,1H),4.5(s,2H) |
| A.48 | n-Propyl | tert.-Butyl | Fp: 54°C |
| A.49 | Cyclopropyl | tert.-Butyl | Fp: 102°C |
| A.50 | n-Butyl | tert.-Butyl | Fp: 49°C |
| A.51 | iso-Propyl | tert.-Butyl | Fp: 110°C |
| A.52 | 2,4-Dichlorphenyl | 4-Fluorphenyl | Fp: 112°C |
| A.53 | 2-Chlorphenyl | 4-Fluorphenyl | Fp: 115°C (Zers.) |
| A.54 | 4-Chlorphenyl | 4-Fluorphenyl | Fp: 125°C (Zers.) |
| A.55 | Phenyl | 4-Fluorphenyl | Fp: 145°C (Zers.) |
| A.56 | n-Butyl | 4-Fluorphenyl | Fp: 78°C |
| A.57 | tert.-Butyl | 2,4-Dichlorphenyl | Fp: 114°C |
| A.58 | 2-Fluorphenyl | 4-Fluorphenyl | Fp: 120°C |
| A.59 | 3-Fluorphenyl | 4-Fluorphenyl | Fp: 149°C |
| A.60 | 4-Fluorphenyl | 4-Fluorphenyl | Fp: 90-4°C |
| A.61 | n-Propyl | 4-Fluorphenyl | Fp: 72°C |
| A.62 | tert.-Butyl | 4-Fluorphenyl | Fp: 131°C |
| A.63 | Cyclopropyl | 4-Fluorphenyl | Fp: 95°C |
| A.64 | Ethyl | 4-Fluorphenyl | Fp: 84°C |
| A.65 | iso-Propyl | 4-Fluorphenyl | Fp: 100°C |

| Nr. | $R^1$ | $R^2$ | Physik. Daten (NMR in $CDCl_3$ [ppm]; TMS als Standard) |
|---|---|---|---|
| A.66 | Ethyl | tert.-Butyl | Fp: 102°C |
| A.67 | Napht-2-yl | 4-Fluorphenyl | Fp: 157-60°C |
| A.68 | Napht-1-yl | 4-Fluorphenyl | Fp: 125°C |
| A.69 | 2,4-Dichlorphenyl | 4-Chlorphenyl | Fp: 90°C (Zers.) |
| A.70 | 4-Fluorphenyl | 4-Fluorbenzyl | Fp: 103°C |
| A.71 | 4-Fluorphenyl | 4-Chlorphenyl | Fp: 105°C |
| A.72 | 4-Chlorphenyl | 4-Fluorbenzyl | Fp: 101°C |
| A.73 | 4-Chlorphenyl | 4-Chlorbenzyl | Fp: 150°C (Zers.) |
| A.74 | 2,4-Dichlorphenyl | 4-Fluorbenzyl | Fp: 41°C |
| A.75 | tert.-Butyl | 4-Fluorbenzyl | Fp: 73°C |
| A.76 | tert.-Butyl | 4-Chlorbenzyl | Fp: 87°C |
| A.77 | 4-Fluorphenyl | α-Methylbenzyl | Fp: 106°C |
| A.78 | 2,4-Dichlorphenyl | 4-Chlorbenzyl | Fp: 77°C |
| A.79 | 2,4-Dichlorphenyl | α-Methylbenzyl | Harz,NMR: 3.2(q,1H),3.4(d,1H),3.6(d,1H), 4.3(d,1H),4.48(d,1H) |
| A.80 | 4-Fluorphenyl | 2,4-Dichlorbenzyl | Fp: 89-91°C |
| A.81 | 4-Chlorphenyl | 2,4-Dichlorbenzyl | Fp: 131°C |
| A.82 | 2,4-Dichlorphenyl | 2,4-Dichlorbenzyl | Fp: 113°C |
| A.83 | tert.-Butyl | 2,4-Dichlorbenzyl | Fp: 84°C |
| A.84 | 4-Fluorphenyl | 2,4-Dichlorphenyl | Fp: 45°C |
| A.85 | 2-Chlorphenyl | 2,4-Dichlorphenyl | Fp: 103°C |
| A.86 | 2,4-Dichlorphenyl | 2,4-Dichlorphenyl | Fp: 52-5°C |
| A.87 | iso-Propyl | 2,4-Dichlorphenyl | Öl,NMR: 3.15(d,1H),3.42(d,1H),4.47(d,1H), 4.92(d,1H) |
| A.88 | 4-Chlorphenyl | 2,4-Dichlorphenyl | Fp: 52-5°C |
| A.89 | 4-Methylphenyl | 2,4-Dichlorphenyl | Fp: 141°C |
| A.90 | 4-Trifluorphenyl | 2,4-Dichlorphenyl | Fp: 122°C |

Tabelle B

Neue 2-(1,2,4-Triazol-1-ylmethyl)-alkene III'

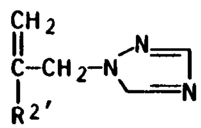

| Nr. | $R^{2'}$ | Physik. Daten (NMR in $CDCl_3$ [ppm]; TMS als Standard) |
|---|---|---|
| B.01 | tert.-Butyl | Öl; NMR: 1.15(s,9H),4.45(s,1H),4.80(s,2H),5.10(s,1H),7.95(s,1H),8.10(s,1H) |
| B.02 | Benzyl | Öl; NMR: 3.3(s,2H),4.7(s,2H),5.0(s,1H),5.1(s,1H),7.0-7.4(s,5H),7.95(s,1H), 8.0(s,1H) |
| B.03 | 4-Fluorbenzyl | NMR: 3.3(s,2H),4.7(s,2H),5.0(s,1H),5.1(s,1H),6.9-7.2(m,4H),8.0(s,2H) |
| B.04 | 4-Chlorbenzyl | NMR: 3.3(s,2H),4.7(s,2H),7.1(d,2H),7.3(d,2H),8.0(2s,2H) |
| B.05 | 2-Methylbenzyl | |
| B.06 | 2,4-Dichlorbenzyl | NMR: 3.4(s,2H),4.75(s,2H),4.9(s,1H),5.05(s,1H),7.0-7.5(m,3H),8.0(s,1H), 8.1(s.1H) |
| B.07 | α-Methylbenzyl | NMR: 1.4(d,3H),3.3(q,1H),4.6(d,2H),5.0(s,1H),5.3(s,1H),7.1-7.4(m,5H), 7.9(s,1H),7.95(s,1H) |

Beispiel 3

3-Cyclopentyl-5-methyl-5-(1,2,4-triazol-1-ylmethyl)-isoxazolin - Salpetersäuresalz

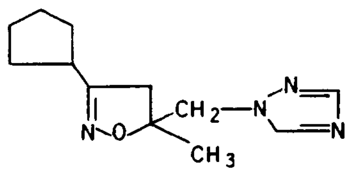

· $HNO_3$

Eine Lösung von 10 g (42,6 mmol) 3-Cyclopentyl-5-methyl-5-(1,2,3-triazol-1-ylmethyl)isoxazolin in 50 ml Methyl-tert.-butylether wurde bei ca. 20°C mit etwa 20 ml konz. Salpetersäure versetzt. Der entstandene Niederschlag wurde anschließend abfiltriert, mit Methyl-tert.-butylether gewaschen und im Exsikkator getrocknet. Ausbeute 24 %; Fp.: 120 bis 122°C; $^1$H-NMR (in $CDCl_3$, TMS als Standard): 1,3 ppm (s, 3H), 1,35-1,8 ppm (m, 8H), 2,65 ppm (m, 1H), 2,85 ppm (d, 1H), 3,1 ppm (d, 1H), 8,3 ppm (s, 1H), 8,9 ppm (s, 1H), 9,75 ppm (s, breit, 1H).

Anwendungsbeispiele (fungizide Wirksamkeit)

Als Vergleichssubstanzen dienten

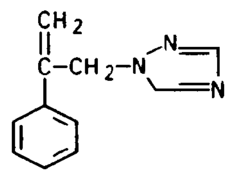

C

bekannt aus der DE-A 28 33 194 (Seite 6, Zeile 28) und

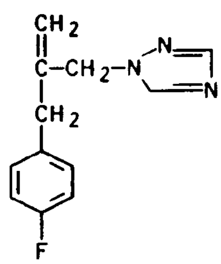

D

bekannt aus der EP-A 94 167 (Verbindung "s", Seite 26).

Beispiel 4

Wirksamkeit gegen Gurkenmehltau

Blätter von in Töpfen gewachsenen Gurkenkeimlingen der Sorte "Chinesische Schlange" wurden im Zweiblattstadium mit einer Sporensuspension des Gurkenmehltaus besprüht. Nach etwa 20 Stunden wurden die Versuchspflanzen mit 0,025 %igen wäßrigen Wirkstoffaufbereitungen, die 80 % Wirkstoff (der Wirkstoffe gemäß den Tabellenbeispielen A.34, A.37, A.38 und A.54) und 20 % Emulgator in der Trockensubstanz enthielten, tropfnaß gesprüht und nach dem Antrocknen des Spritzbelages im Gewächshaus bei Temperaturen zwischen 20 und 22°C und 70 bis 80 % relativer Luffeuchtigkeit aufgestellt.

Nach 21 Tagen wurde das Ausmaß des Pilzbefalls beurteilt.

Gegenüber einem Kontrollversuch (keine Behandlung, 100 % Pilzbefall) und den bekannten Vergleichsverbindungen C und D (50 % bzw. 100 % Pilzbefall) zeigten die Substanzen A.34, A.37, A.38 und A.54 eine sehr gute fungizide Wirkung (0 bis 5 % Pilzbefall).

Beispiel 5

Wirksamkeit gegen Pyrenophora teres

Gerstenkeimlinge der Sorte "Igri" wurden im Zweiblattstadium mit 0,05 %igen wäßrigen Suspensionen, die 80 % Wirkstoff (der Wirkstoffe gemäß den Tabellenbeispielen A.39, A.53, A.54 und A.58) und 20 % Emulgator in der Trockensubstanz enthielten, tropfnaß gespritzt. Nach 24 Stunden wurden die Pflanzen mit einer Sporensuspension des Pilzes Pyrenophora teres infiziert und für 48 Stunden in eine Klimakammer mit hoher Luftfeuchtigkeit bei 18°C gestellt.

Anschließend wurden die Pflanzen im Gewächshaus bei 20 bis 22°C und 70 % relativer Luftfeuchtigkeit für weitere 5 Tage kultiviert. Danach wurde das Ausmaß des Pilzbefalls beurteilt.

Gegenüber einem Kontrollversuch (keine Behandlung, 60 % Pilzbefall) und der bekannten Vergleichsverbindung D (40 % Pilzbefall) zeigte sich, daß die behandelten Pflanzen nur einen Pilzbefall von 0-5 % hatten.

## Patentansprüche

### Patentansprüche für folgende Vertragsstaaten : AT, BE, CH, DE, DK, FR, GB, GR, IT, LI, NL, SE

1. 5-(1,2,4-Triazol-1-ylmethyl)-isoxazoline der allgemeinen Formel I

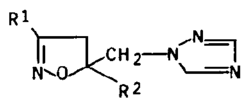

I

in der die Substituenten $R^1$ und $R^2$ die folgende Bedeutung haben:
Wasserstoff, eine $C_1$-$C_4$-Alkoxy-$C_1$-$C_4$-alkylgruppe, eine partiell oder vollständig halogenierte $C_1$-$C_6$-Alkylgruppe, eine $C_1$-$C_6$-Alkylgruppe, die einen $C_3$-$C_8$-Cycloalkyl-, Phenyl- oder Naphthylrest tragen kann, eine $C_3$-$C_8$-Cycloalkyl-, Phenyl- oder Naphthylgruppe oder eine $C_5$-$C_6$-Heteroarylgruppe, die 3 Stickstoffatome, von denen max. 2 benachbart sein dürfen, oder bis zu 2 der folgenden Heteroatome enthält: Sauerstoff, Schwefel und/oder Stickstoff, wobei jeweils 2 Sauerstoff- und/oder Schwefelatome nicht benachbart sein dürfen, und wobei die Phenyl-, Naphthyl- und Heteroarylteile der genannten Gruppen noch jeweils einen Phenyl- oder Phenoxyrest mit bis zu 3 Halogen-substituenten oder bis zu 3 der folgenden Reste tragen können: Halogen, Cyano, Nitro, $C_1$-$C_4$-Alkyl, partiell oder vollständig halogeniertes $C_1$-$C_4$-Alkyl oder $C_1$-$C_4$-Alkoxy,
sowie die pflanzenverträglichen mineralsauren Salze und Metallkomplexe von I.

2. Verfahren zur Herstellung der 5-(1,2,4-Triazol-1-ylmethyl)-isoxazoline I gemäß Anspruch 1, dadurch gekennzeichnet, daß man ein Aldehydoxim der Formel IIa

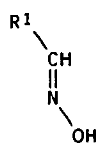

IIa

reduktiv in ein Nitriloxid der Formel IIb

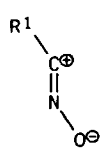

IIb

überführt, dieses mit einem 2-(1,2,4-Triazol-1-ylmethyl)alken der Formel III

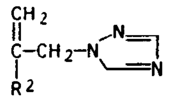

III

umsetzt und die erhaltenen Verbindungen gewünschtenfalls in ihre pflanzenverträglichen Salze oder Metallkomplexe überführt.

3. Verwendung der 5-(1,2,4-Triazol-1-ylmethyl)-isoxaline I, deren pflanzenverträglichen Salzen und Metallkomplexen gemäß Anspruch 1 als Fungizide im Pflanzenschutz.

4. Fungizides Mittel, enthaltend mindestens ein 5-(1,2,4-Triazol-1-yl-methyl)-isoxazolin der Formel I, dessen pflanzenverträgliches Salz oder dessen Metallkomplex gemäß Anspruch 1 und einen flüssigen und/oder festen Trägerstoff.

5. Verfahren zur Bekämpfung von Pilzen, dadurch gekennzeichnet, daß man eine fungizid wirksame Menge eines 5-(1,2,4-Triazol-1-ylmethyl)-isoxazolin der Formel I, von dessen pflanzenverträglichen Salzen oder Metallkomplexen gemäp Anspruch 1, auf Pilze, vom Pilzbefall bedrohte Materialien, Pflanzen, deren Lebensraum oder auf das Saatgut der bedrohten Pflanzen einwirken läßt.

6. Verwendung der 5-(1,2,4-Triazol-1-ylmethyl)-isoxazoline I, deren pflanzenverträglichen Salzen und Metallkomplexen gemäß Anspruch 1 zur Regulierung des Pflanzenwachstums.

7. Mittel zur Regulierung des Pflanzenwachstums, enthaltend mindestens ein 5-(1,2,4-Triazol-1-ylmethyl)-isoxazolin der Formel I, dessen pflanzenverträgliches Salz oder dessen Metallkomplex gemäp Anspruch 1 und einen flüssigen und/oder festen Trägerstoff.

8. Verfahren zur Regulierung des Pflanzenwachstums, dadurch gekennzeichnet, daß man eine zum Regulieren des Pflanzenwachstums wirksame Menge eines 5-(1,2,4-Triazol-1-ylmethyl)-isoxazolins der Formel I, von dessen pflanzenverträglichen Salzen oder Metallkomplexen gemäß Anspruch 1, auf die Pflanzen, deren Lebensraum oder auf das Saatgut der Pflanzen einwirken läßt.

9. 2-(1,2,4-Triazol-1-ylmethyl)-alkene der allgemeinen Formel III′

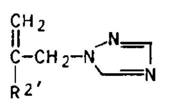

III′

in der der Substituent $R^{2'}$ die folgende Bedeutung hat: eine $C_1$-$C_4$-Alkoxy-$C_1$-$C_4$-alkylgruppe, eine partiell oder vollständig halogenierte $C_1$-$C_6$-Alkylgruppe, eine $C_1$-$C_6$-Alkylgruppe, die einen $C_3$-$C_8$-Cycloalkyl-, Phenyl- oder Naphthylrest tragen kann, eine $C_3$-$C_8$-Cycloalkyl- oder Naphthylgruppe oder eine $C_5$-$C_6$-Heteroarylgruppe, die 3 Stickstoffatome, von denen max. 2 benachbart sein dürfen, oder bis zu 2 der folgenden Heteroatome enthält: Sauerstoff, Schwefel und/oder Stickstoff, wobei jeweils 2 Sauerstoff- und/oder Schwefelatome nicht benachbart sein dürfen, und wobei die Phenyl-, Naphthyl- und Heteroarylteile der genannten Gruppen noch jeweils einen Phenyl- oder Phenoxyrest mit bis zu 3 Halogensubstituenten oder bis zu 3 der folgenden Reste tragen können: Halogen, Cyano, Nitro, $C_1$-$C_4$-Alkyl, partiell oder vollständig halogeniertes $C_1$-$C_4$-Alkyl oder $C_1$-$C_4$-Alkoxy,
ausgenommen Methyl.

10. 2-(1,2,4-Triazol-1-ylmethyl)-3,3-dimethyl-but-1-en.

11. Verbindung gemäß Anspruch 1, nämlich 3-(4-Fluorphenyl)-5-(4-fluorphenyl)-5-(1,2,4-triazol-1-ylmethyl)-isoxazolin.

**Patentansprüche für folgenden Vertragsstaat : ES**

1. Fungizides Pflanzenschutzmittel, enthaltend einen flüssigen und/oder festen Trägerstoff und eine fungizid wirksame Menge eines 5-(1,2,4-Triazol-1-ylmethyl)-isoxazolins der allgemeinen Formel I,

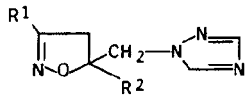

I

in der die Substituenten $R^1$ und $R^2$ die folgende Bedeutung haben:
Wasserstoff, eine $C_1$-$C_4$-Alkoxy-$C_1$-$C_4$-alkylgruppe, eine partiell oder vollständig halogenierte $C_1$-$C_6$-Alkylgruppe, eine $C_1$-$C_6$-Alkylgruppe, die einen $C_3$-$C_8$-Cycloalkyl-, Phenyl- oder Naphthylrest tragen kann, eine $C_3$-$C_8$-Cycloalkyl-, Phenyl- oder Naphthylgruppe oder eine $C_5$-$C_6$-Heteroarylgruppe, die 3 Stickstoffatome, von denen max. 2 benachbart sein dürfen, oder bis zu 2 der folgenden Heteroatome enthält: Sauerstoff, Schwefel und/oder Stickstoff, wobei jeweils 2 Sauerstoff- und/oder Schwefelatome nicht benachbart sein dürfen, und wobei die Phenyl-, Naphthyl- und Heteroarylteile der genannten Gruppen noch jeweils einen Phenyl- oder Phenoxyrest mit bis zu 3 Halogen-substituenten oder bis zu 3 der folgenden Reste tragen können: Halogen, Cyano, Nitro, $C_1$-$C_4$-Alkyl, partiell oder vollständig halogeniertes $C_1$-$C_4$-Alkyl oder $C_1$-$C_4$-Alkoxy,
dessen pflanzenverträgliches Salz oder dessen Metallkomplex.

2. Fungizides Pflanzenschutzmittel, enthaltend einen flüssigen und/oder festen Trägerstoff und 3-(4-Fluorphenyl)-5-(4-fluorphenyl)-5-(1,2,4-triazol-1-ylmethyl)-isoxazolin.

3. Mittel zur Regulierung des Pflanzenwachstums, enthaltend einen flüssigen und/oder festen Trägerstoff und mindestens ein 5-(1,2,4-Triazol-1-ylmethyl)-isoxazolin der allgemeinen Formel I,

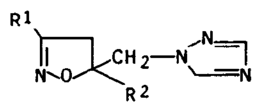

I

in der die Substituenten $R^1$ und $R^2$ die folgende Bedeutung haben:
Wasserstoff, eine $C_1$-$C_4$-Alkoxy-$C_1$-$C_4$-alkylgruppe, eine partiell oder vollständig halogenierte $C_1$-$C_6$-Alkylgruppe, eine $C_1$-$C_6$-Alkylgruppe, die einen $C_3$-$C_8$-Cycloalkyl-, Phenyl- oder Naphthylrest tragen kann, eine $C_3$-$C_8$-Cycloalkyl-, Phenyl- oder Naphthylgruppe oder eine $C_5$-$C_6$-Heteroarylgruppe, die 3 Stickstoffatome, von denen max. 2 benachbart sein dürfen, oder bis zu 2 der folgenden Heteroatome enthält: Sauerstoff, Schwefel und/oder Stickstoff, wobei jeweils 2 Sauerstoff- und/oder Schwefelatome nicht benachbart sein dürfen, und wobei die Phenyl-, Naphthyl- und Heteroarylteile der genannten Gruppen noch jeweils einen Phenyl- oder Phenoxyrest mit bis zu 3 Halogen-substituenten oder bis zu 3 der folgenden Reste tragen können: Halogen, Cyano, Nitro, $C_1$-$C_4$-Alkyl, partiell oder vollständig halogeniertes $C_1$-$C_4$-Alkyl oder $C_1$-$C_4$-Alkoxy,
dessen pflanzenverträgliches Salz oder dessen Metallkomplex.

4. Verfahren zur Herstellung der 5-(1,2,4-Triazol-1-ylmethyl)-isoxazoline I gemäß Anspruch 1 oder 3, dadurch gekennzeichnet, daß man ein Aldehydoxim der Formel IIa

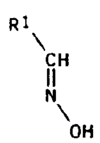

IIa

reduktiv in ein Nitriloxid der Formel IIb

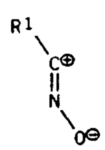

IIb

überführt, dieses mit einem 2-(1,2,4-Triazol-1-ylmethyl)alken der Formel III

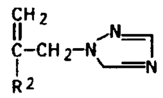

III

umsetzt und die erhaltenen Verbindungen gewünschtenfalls in ihre pflanzenverträglichen Salze oder Metallkomplexe überführt.

5. Verfahren zur Bekämpfung von Pilzen, dadurch gekennzeichnet, daß man eine fungizid wirksame Menge eines 5-(1,2,4-Triazol-1-ylmethyl)-isoxazolin der Formel I, von dessen pflanzenverträglichen Salzen oder Metallkomplexen gemäp Anspruch 1, auf Pilze, vom Pilzbefall bedrohte Materialien, Pflanzen, deren Lebensraum oder auf das Saatgut der bedrohten Pflanzen einwirken läßt.

6. Verfahren zur Regulierung des Pflanzenwachstums, dadurch gekennzeichnet, daß man eine zum Regulieren des Pflanzenwachstums wirksame Menge eines 5-(1,2,4-Triazol-1-ylmethyl)-isoxazolins der Formel I, von dessen pflanzenverträglichen Salzen oder Metallkomplexen gemäß D Anspruch 3, auf die Pflanzen, deren Lebensraum oder auf das Saatgut der Pflanzen einwirken läßt.

7. Verfahren zur Herstellung von 2-(1,2,4-Triazol-1-ylmethyl)-alkenen der allgemeinen Formel III'

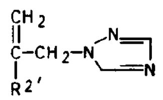

III'

in der der Substituent $R^{2'}$ die folgende Bedeutung hat: eine $C_1$-$C_4$-Alkoxy-$C_1$-$C_4$-alkylgruppe, eine partiell oder vollständig halogenierte $C_1$-$C_6$-Alkylgruppe, eine $C_1$-$C_6$-Alkylgruppe, die einen $C_3$-$C_8$-Cycloalkyl-, Phenyl- oder Naphthylrest tragen kann, eine $C_3$-$C_8$-Cycloalkyl- oder Naphthylgruppe oder eine $C_5$-$C_6$-Heteroarylgruppe, die 3 Stickstoffatome, von denen max. 2 benachbart sein dürfen, oder bis zu 2 der folgenden Heteroatome enthält: Sauerstoff, Schwefel und/oder Stickstoff, wobei jeweils 2 Sauerstoff- und/oder Schwefelatome nicht benachbart sein dürfen, und wobei die Phenyl-, Naphthyl- und Heteroarylteile der genannten Gruppen noch jeweils einen Phenyl- oder Phenoxyrest mit bis zu 3 Halogensubstituenten oder bis zu 3 der folgenden Reste tragen können: Halogen, Cyano, Nitro, $C_1$-$C_4$-Alkyl, partiell oder vollständig halogeniertes $C_1$-$C_4$-Alkyl oder $C_1$-$C_4$-Alkoxy,
ausgenommen Methyl,
dadurch gekennzeichnet, daß man Methylen-phosphoniumylide IV

$$(R)_3P{=}CH_2 \qquad IV$$

wobei R einen aromatischen oder $C_6$-cycloaliphatischen Rest bedeutet, mit 1,2,4-Triazol-1-ylmethyl-ketonen V

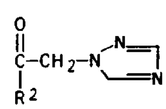

V

umsetzt.

8. Verfahren zur Herstellung von 2-(1,2,4-Triazol-1-ylmethyl)-alkenen der allgemeinen Formel III'

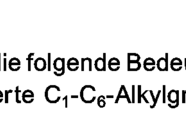

III'

in der der Substituent $R^{2'}$ die folgende Bedeutung hat: eine $C_1$-$C_4$-Alkoxy-$C_1$-$C_4$-alkylgruppe, eine partiell oder vollständig halogenierte $C_1$-$C_6$-Alkylgruppe, eine $C_1$-$C_6$-Alkylgruppe, die einen $C_3$-$C_8$-Cycloalkyl-, Phenyl- oder Naphthylrest tragen kann, eine $C_3$-$C_8$-Cycloalkyl- oder Naphthylgruppe oder eine $C_5$-$C_6$-Heteroarylgruppe, die 3 Stickstoffatome, von denen max. 2 benachbart sein dürfen, oder bis zu 2 der folgenden Heteroatome enthält: Sauerstoff, Schwefel und/oder Stickstoff, wobei jeweils 2 Sauerstoff- und-/oder Schwefelatome nicht benachbart sein dürfen, und wobei die Phenyl-, i Naphthyl- und Heteroarylteile der genannten Gruppen noch jeweils einen Phenyl- oder Phenoxyrest mit bis zu 3 Halogensubstituenten oder bis zu 3 der folgenden Reste tragen können: Halogen, Cyano, Nitro, $C_1$-$C_4$-Alkyl, partiell oder vollständig halogeniertes $C_1$-$C_4$-Alkyl oder $C_1$-$C_4$-Alkoxy,
ausgenommen Methyl,
dadurch gekennzeichnet, daß man Halogenmethyl-alkene VI′

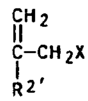

VI′

wobei X Halogen bedeutet,
in Gegenwart einer Base mit 1,2,4-Triazol umsetzt.

## Claims

### Claims for the following Contracting States : AT, BE, CH, DE, DK, FR, GB, GR, IT, LI, NL, SE

**1.** A 5-(1,2,4-triazol-1-ylmethyl)-isoxazoline of the general formula I

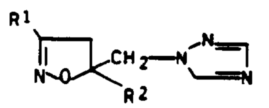

I

where $R^1$ and $R^2$ are each hydrogen, $C_1$-$C_4$-alkoxy-$C_1$-$C_4$-alkyl, partially or completely halogenated $C_1$-$C_6$-alkyl or $C_1$-$C_6$-alkyl which may carry a $C_3$-$C_8$-cycloalkyl, phenyl or naphthyl radical, or are each $C_3$-$C_8$-cycloalkyl, phenyl or naphthyl, or a $C_5$- or $C_6$-hetaryl group which contains 3 nitrogen atoms, not more than 2 of which may be adjacent, or up to 2 of the following hetero atoms: oxygen, sulfur or nitrogen, where 2 oxygen or sulfur atoms may not be adjacent, and where the phenyl, naphthyl and hetaryl moieties of the stated groups may furthermore each carry a phenyl or phenoxy radical having up to 3 halogen substituents, or up to 3 of the following radicals: halogen, cyano, nitro, $C_1$-$C_4$-alkyl, partially or completely halogenated $C_1$-$C_4$-alkyl or $C_1$-$C_4$-alkoxy, or a plant-tolerated mineral acid salt or metal complex of I.

**2.** A process for the preparation of a 5-(1,2,4-triazol-1-ylmethyl)-isoxazoline I as claimed in claim 1, wherein an aldehyde oxime of the formula IIa

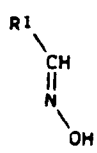

IIa

is converted with reduction into a nitrile oxide of the formula IIb

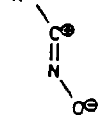

IIb

and the latter is reacted with a 2-(1,2,4-triazol-1-ylmethyl)-alkene of the formula III

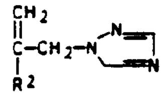

III

and the resulting compound is, if desired, converted into a plant-tolerated salt or metal complex thereof.

**3.** Use of a 5-(1,2,4-triazol-1-ylmethyl)-isoxazoline I or a plant-tolerated salt or metal complex thereof as claimed in claim 1 as a fungicide in plant protection.

**4.** A fungicide containing one or more 5-(1,2,4-triazol-1-ylmethyl)-isoxazolines of the formula I or a plant-tolerated salt or metal complex thereof as claimed in claim 1 and a liquid or solid carrier.

**5.** A method for controlling fungi, wherein a fungicidal amount of a 5-(1,2,4-triazol-1-ylmethyl)-isoxazoline of the formula I, of a plant-tolerated salt or of a metal complex thereof as claimed in claim 1 is allowed to act on fungi, on materials threatened by fungal attack, on plants, on their habitat or on the seed of the threatened plants.

**6.** Use of a 5-(1,2,4-triazol-1-ylmethyl)-isoxazoline I or a plant-tolerated salt or metal complex thereof as claimed in claim 1 for regulating plant growth.

**7.** A plant growth regulator containing one or more 5-(1,2,4-triazol-1-ylmethyl)-isoxazolines of the formula I or a plant-tolerated salt or metal complex thereof as claimed in claim 1 and a liquid or solid carrier.

**8.** A method for regulating plant growth, wherein an amount, effective for regulating plant growth, of a 5-(1,2,4-triazol-1-ylmethyl)-isoxazoline of the formula I or a plant-tolerated salt or metal complex thereof as claimed in claim 1 is allowed to act on the plants, on their habitat or on the seed of the plants.

**9.** A 2-(1,2,4-triazol-1-ylmethyl)-alkene of the formula III′

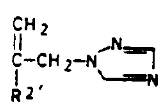

III′

where $R^{2'}$ is $C_1$-$C_4$-alkoxy-$C_1$-$C_4$-alkyl, partially or completely halogenated $C_1$-$C_6$-alkyl or $C_1$-$C_6$-alkyl which may carry a $C_3$-$C_8$-cycloalkyl, phenyl or naphthyl radical, or a $C_3$-$C_8$-cycloalkyl or naphthyl, or a $C_5$- or $C_6$-hetaryl group which contains 3 nitrogen atoms, not more than two of which may be adjacent, or up to 2 of the following hetero atoms: oxygen, sulfur or nitrogen, where 2 oxygen or sulfur atoms may not be adjacent, and where the phenyl, naphthyl and hetaryl moieties of the stated groups may furthermore each carry a phenyl or phenoxy radical having up to 3 halogen substituents, or up to 3 of the following radicals: halogen, cyano, nitro, $C_1$-$C_4$-alkyl, partially or completely halogenated $C_1$-$C_4$-alkyl or $C_1$-$C_4$-alkoxy, with the exception of methyl.

**10.** 2-(1,2,4-Triazol-1-ylmethyl)-3,3-dimethylbut-1-ene.

**11.** A compound as claimed in claim 1, namely 3-(4-fluorophenyl)-5-(4-fluorophenyl)-5-(1,2,4-triazol-1-ylmethyl)-isoxazoline.

**Claims for the following Contracting State : ES**

**1.** A fungicidal crop protection agent containing a liquid or solid carrier and a fungicidal amount of a 5-(1,2,4-triazol-1-ylmethyl)-isoxazoline of the general formula I

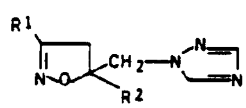

I

where $R^1$ and $R^2$ are each hydrogen, $C_1$-$C_4$-alkoxy-$C_1$-$C_4$-alkyl, partially or completely halogenated $C_1$-$C_6$-alkyl or $C_1$-$C_6$-alkyl which may carry a $C_3$-$C_8$-cycloalkyl, phenyl or naphthyl radical, or are each $C_3$-$C_8$-cycloalkyl, phenyl or naphthyl, or a $C_5$- or $C_6$-hetaryl group which contains 3 nitrogen atoms, not more than 2 of which may be adjacent, or up to 2 of the following hetero atoms: oxygen, sulfur or nitrogen, where 2 oxygen or sulfur atoms may not be adjacent, and where the phenyl, naphthyl and hetaryl moieties of the stated groups may furthermore each carry a phenyl or phenoxy radical having up to 3 halogen substituents, or up to 3 of the following radicals: halogen, cyano, nitro, $C_1$-$C_4$-alkyl, partially or completely halogenated $C_1$-$C_4$-alkyl or $C_1$-$C_4$-alkoxy, or a plant-tolerated salt or metal complex thereof.

**2.** A fungicidal crop protection agent containing a liquid or solid carrier and 3-(4-fluorophenyl)-5-(4-fluorophenyl)-5-(1,2,4-triazol-1-ylmethyl)-isoxazoline.

**3.** A plant growth regulator containing a liquid or solid carrier and one or more 5-(1,2,4-triazol-1-yl-methyl)-isoxazolines of the general formula I.

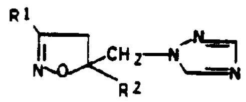

I

where $R^1$ and $R^2$ are each hydrogen, $C_1$-$C_4$-alkoxy-$C_1$-$C_4$-alkyl, partially or completely halogenated $C_1$-$C_6$-alkyl or $C_1$-$C_6$-alkyl which may carry a $C_3$-$C_8$-cycloalkyl, phenyl or naphthyl radical, or are each $C_3$-$C_8$-cycloalkyl, phenyl or naphthyl, or a $C_5$- or $C_6$-hetaryl group which contains 3 nitrogen atoms, not more than 2 of which may be adjacent, or up to 2 of the following hetero atoms: oxygen, sulfur or nitrogen, where 2 oxygen or sulfur atoms may not be adjacent, and where the phenyl, naphthyl and hetaryl moieties of the stated groups may furthermore each carry a phenyl or phenoxy radical having up to 3 halogen substituents, or up to 3 of the following radicals: halogen, cyano, nitro, $C_1$-$C_4$-alkyl, partially or completely halogenated $C_1$-$C_4$-alkyl or $C_1$-$C_4$-alkoxy, or a plant-tolerated salt or metal complex thereof.

**4.** A process for the preparation of a 5-(1,2,4-triazol-1-ylmethyl)-isoxazoline I as claimed in claim 1 or 3, wherein an aldehyde oxime of the formula IIa

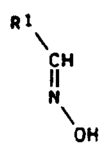

IIa

is converted with reduction into a nitrile oxide of the formula IIb

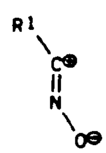

IIb

and the latter is reacted with a 2-(1,2,4-triazol-1-ylmethyl)-alkene of the formula III

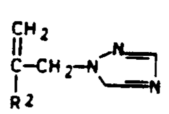

III

and the resulting compound is, if desired, converted into a plant-tolerated salt or metal complex thereof.

**5.** A method for controlling fungi, wherein a fungicidal amount of a 5-(1,2,4-triazol-1-ylmethyl)-isoxazoline of the formula I or a plant-tolerated salt or metal complex thereof as claimed in claim 1 is allowed to act on fungi, on materials threatened by fungal attack, on plants, on their habitat or on the seed of the threatened plants.

6. A method for regulating plant growth, wherein an amount, effective for regulating plant growth, of a 5-(1,2,4-triazol-1-ylmethyl)-isoxazoline of the formula I or a plant-tolerated salt or metal complex thereof as claimed in claim 3 is allowed to act on the plants, on their habitat or on the seed of the plants.

7. A process for the preparation of a 2-(1,2,4-triazol-1-ylmethyl)-alkene of the formula III′

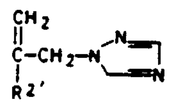

III′

where $R^{2'}$ is $C_1$-$C_4$-alkoxy-$C_1$-$C_4$-alkyl, partially or completely halogenated $C_1$-$C_6$-alkyl or $C_1$-$C_6$-alkyl which may carry a $C_3$-$C_8$-cycloalkyl, phenyl or naphthyl radical, or $C_3$-$C_8$-cycloalkyl or naphthyl, or a $C_5$- or $C_6$-hetaryl group which contains 3 nitrogen atoms, not more than two of which may be adjacent, or up to 2 of the following hetero atoms: oxygen, sulfur or nitrogen, where 2 oxygen or sulfur atoms may not be adjacent, and where the phenyl, naphthyl and hetaryl moieties of the stated groups may furthermore each carry a phenyl or phenoxy radical having up to 3 halogen substituents, or up to 3 of the following radicals: halogen, cyano, nitro, $C_1$-$C_4$-alkyl, partially or completely halogenated $C_1$-$C_4$-alkyl or $C_1$-$C_4$-alkoxy, with the exception of methyl,
wherein a methylenephosphoniumylide IV

$(R)_3P{=}CH_2$ IV

where R is an aromatic or $C_6$-cycloaliphatic radical, is reacted with a 1,2,4-triazol-1-ylmethyl ketone V

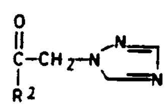

V

8. A process for the preparation of a 2-(1,2,4-triazol-1-ylmethyl)-alkene of the general formula III′

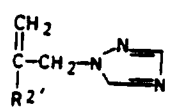

III′

where $R^{2'}$ is $C_1$-$C_4$-alkoxy-$C_1$-$C_4$-alkyl, partially or completely halogenated $C_1$-$C_6$-alkyl or $C_1$-$C_6$-alkyl which may carry a $C_3$-$C_8$-cycloalkyl, phenyl or naphthyl radical, or $C_3$-$C_8$-cycloalkyl or naphthyl, or a $C_5$- or $C_6$-hetaryl group which contains 3 nitrogen atoms, not more than two of which may be adjacent, or up to 2 of the following hetero atoms: oxygen, sulfur or nitrogen, where 2 oxygen or sulfur atoms may not be adjacent, and where the phenyl, naphthyl and hetaryl moieties of the stated groups may furthermore each carry a phenyl or phenoxy radical having up to 3 halogen substituents, or up to 3 of the following radicals: halogen, cyano, nitro, $C_1$-$C_4$-alkyl, partially or completely halogenated $C_1$-$C_4$-alkyl or $C_1$-$C_4$-alkoxy, with the exception of methyl,
wherein a halomethylalkene VI′

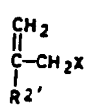

VI′

where X is halogen, is reacted with 1,2,4-triazole in the presence of a base.

## Revendications

### Revendications pour les Etats contractants suivants : AT, BE, CH, DE, DK, FR, GB, GR, IT, LI, NL, SE

1. 5-(1,2,4-triazole-1-ylméthyle)-isoxazolines de la formule générale I

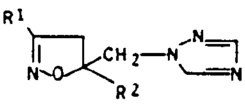

I

dans laquelle les substituants $R^1$ et $R^2$ ont les significations suivantes:
hydrogène, un groupe alcoxy en C1-C4-alkyle en C1-C4, un groupe alkyle en C1-C6 partiellement ou totalement halogéné, un groupe alkyle en C1-C6 qui peut porter un reste cycloalkyle en C3-C8 phényle ou naphtyle, un groupe cycloalkyle en C3-C8, phényle ou naphtyle ou un groupe hétéroaryle en C5-C6 qui contient 3 atomes d'azote dont au maximum 2 doivent être voisins, ou jusqu'à deux des hétéroatomes suivants : oxygène, soufre et/ou azote, 2 atomes d'oxygène et/ou soufre ne devant chaque fois pas être voisins, et les parties phényle, naphtyle et hétéroaryle des groupes indiqués pouvant encore porter chacun un reste phényle ou phénoxy ayant jusqu'à 3 substituants halogène ou jusqu'à 3 des restes suivants : halogène, cyano, nitro, alkyle en C1-C4 ou alcoxy en C1-C4 partiellement ou totalement halogénés ainsi que les sels d'acides minéraux et complexes métalliques de I acceptables pour les plantes.

2. Procédé de préparation des 5-(1,2,4-triazole-1-ylméthyle)-isoxazolines de la formule générale I selon la revendication 1, caractérisé par le fait que l'on transforme une aldéhydoxyme de la formule IIa

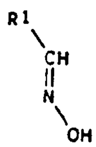

IIa

par réduction, en un nitriloxyde de la formule IIb

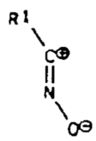

IIb

on fait réagir ce dernier avec un 2-(1,2,4-triazol-1-ylméthyl)alcène de la formule III

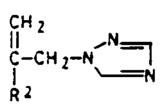

III

et on transforme les composés obtenus, éventuellement, en leurs sels ou complexes métalliques acceptables par les plantes.

3. Utilisation des 5-(1,2,4-triazole-1-ylméthyle)-isoxazolines de la formule générale I, leurs sels et complexes métalliques acceptables par les plantes selon la revendication 1, comme fongicides pour la protection des plantes.

4. Agent fongicide contenant au moins un 5-(1,2,4-triazole-1-ylméthyle)-isoxazoline de la formule générale I ses sels et complexes métalliques acceptables par les plantes selon la revendication 1 et un véhicule liquide et/ou solide.

5. Procédé de lutte contre les champignons, caractérisé par le fait que l'on fait agir sur les champignons, les matériaux, plantes et leur biotope, menacés d'une attaque par les champignons ou sur les semences des plantes menacées, une quantité active comme fongicide d'un 5-(1,2,4-triazole-1-ylméthyle)-isoxazoline de formule générale I ses sels et complexes métalliques acceptables par les plantes selon la revendication 1.

6. Utilisation de 5-(1,2,4-triazole-1-ylméthyle)-isoxazolines de la formule générale I, leurs sels et complexes métalliques acceptables par les plantes selon la revendication 1 pour régulariser la croissance des plantes.

7. Moyen de régularisation de la croissance des plantes contenant au moins un 5-(1,2,4-triazole-1-ylméthyle)-isoxazoline de formule générale I, ses sels et complexes métalliques acceptables par les plantes selon la revendication 1 et un véhicule liquide et/ou solide.

8. Procédé de régularisation de la croissance des plantes, caractérisé par le fait que l'on fait agir une quantité efficace pour régulariser la croissance des plantes d'un 5-(1,2,4-triazole-1-ylméthyle)-isoxazoline de formule générale I, ses sels et complexes métalliques acceptables sur les plantes, leur biotope ou sur la semence des plantes.

9. 2-(1,2,4-triazole-1-yl-méthyl)-alcène de la formule générale III'

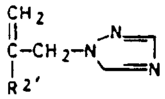

III'

dans laquelle le substituant $R^{2'}$ a les significations suivantes: un groupe alcoxy en C1-C4-alkyle en C1-C4, un groupe alkyle en C1-C6 partiellement ou totalement halogéné, un groupe alkyle en C1-C6 qui peut porter un reste cycloalkyle en C3-C8 phényle ou naphtyle, un groupe cycloalkyle en C3-C8, phényle ou naphtyle ou un groupe hétéroaryle en C5-C6 qui contient 3 atomes d'azote dont au maximum 2 doivent être voisins, ou jusqu'à deux des hétéroatomes suivants : oxygène, soufre et/ou azote, 2 atomes d'oxygène et/ou soufre ne devant chaque fois pas être voisins, et les parties phényle, naphtyle et hétéroaryle des groupes indiqués pouvant encore porter chacun un reste phényle ou phénoxy ayant jusqu'à 3 substituants halogène ou jusqu'à 3 des restes suivants : halogène, cyano, nitro, alkyle en C1-C4 ou alcoxy en C1-C4 partiellement ou totalement halogénés - excepté méthyle.

10. 2-(1,2,4-triazol-1-ylméthyl)-3,3-diméthyl-but-1-ène.

11. Composé selon la revendications 1, à savoir 3-(4-fluorophényl)-5-(4-fluorophényl)-5-(1,2,4-triazol-1-ylméthyl)-isoxazoline.

**Revendications pour l'Etat contractant suivant : ES**

1. Agent de protection des plantes fongicide contenant un véhicule liquide et/ou solide et une quantité active du point de vue fongicide d'un 5-(1,2,4-triazole-1-ylméthyle)-isoxazoline de la formule générale I

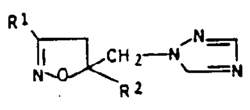

I

dans laquelle les substituants $R^1$ et $R^2$ ont les significations suivantes:
hydrogène, un groupe alcoxy en C1-C4-alkyle en C1-C4, un groupe alkyle en C1-C6 partiellement ou totalement halogéné, un groupe alkyle en C1-C6 qui peut porter un reste cycloalkyle en C3-C8 phényle ou naphtyle, un groupe cycloalkyle en C3-C8, phényle ou naphtyle ou un groupe hétéroaryle en C5-C6 qui contient 3 atomes d'azote dont au maximum 2 doivent être voisins, ou jusqu'à deux des hétéroatomes suivants : oxygène, soufre et/ou azote, 2 atomes d'oxygène et/ou soufre ne devant chaque fois pas être voisins, et les parties phényle, naphtyle et hétéroaryle des groupes indiqués pouvant encore porter chacun un reste phényle ou phénoxy ayant jusqu'à 3 substituants halogène ou jusqu'à 3 des restes suivants :

halogène, cyano, nitro, alkyle en C1-C4 ou alcoxy en C1-C4 partiellement ou totalement halogénés ainsi que son sel et complexe métallique de I acceptable pour les plantes.

2. Agent de protection des plantes fongicide contenant un véhicule liquide et/ou solide et le 3-(4-fluorophényl)-5-(4-fluorophényl)-5-(1,2,4-triazol-1-ylméthyl)-isoxazoline.

3. Moyen de régularisation de la croissance des plantes contenant un véhicule liquide et/ou solide et au moins un 5-(1,2,4-triazole-1-ylméthyle)-isoxazoline de la formule générale I

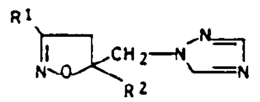

I

dans laquelle les substituants $R^1$ et $R^2$ ont les significations suivantes:
hydrogène, un groupe alcoxy en C1-C4-alkyle en C1-C4, un groupe alkyle en C1-C6 partiellement ou totalement halogéné, un groupe alkyle en C1-C6 qui peut porter un reste cycloalkyle en C3-C8 phényle ou naphtyle, un groupe cycloalkyle en C3-C8, phényle ou naphtyle ou un groupe hétéroaryle en C5-C6 qui contient 3 atomes d'azote dont au maximum 2 doivent être voisins, ou jusqu'à deux des hétéroatomes suivants : oxygène, soufre et/ou azote, 2 atomes d'oxygène et/ou soufre ne devant chaque fois pas être voisins, et les parties phényle, naphtyle et hétéroaryle des groupes indiqués pouvant encore porter chacun un reste phényle ou phénoxy ayant jusqu'à 3 substituants halogène ou jusqu'à 3 des restes suivants : halogène, cyano, nitro, alkyle en C1-C4 ou alcoxy en C1-C4 partiellement ou totalement halogénés ainsi que son sel et complexe métallique de I acceptable pour les plantes.

4. Procédé de préparation des 5-(1,2,4-triazole-1-ylméthyle)-isoxazolines de la formule générale I selon la revendication 1, caractérisé par le fait que l'on transforme une aldéhydoxyme de la formule IIa

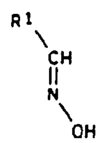

IIa

par réduction, en un nitriloxyde de la formule IIb

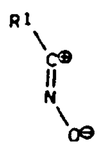

IIb

on fait réagir ce dernier avec un 2-(1,2,4-triazol-1-ylméthyl)alcène de la formule III

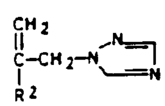

III

et on transforme les composés obtenus, éventuellement, en leurs sels ou complexes métalliques acceptables par les plantes.

5. Procédé de lutte contre les champignons, caractérisé par le fait que l'on fait agir sur les champignons, les matériaux, plantes et leur biotope, menaces d'une attaque par les champignons ou sur les semences des plantes menacées, une quantité active comme fongicide d'un 5-(1,2,4-triazole-1-ylméthyle)-isoxazoline de formule générale I ses sels et complexes métalliques acceptables par les plantes selon la revendication 1.

6. Procédé de régularisation de la croissance des plantes, caractérisé par le fait que l'on fait agir une quantité efficace pour régulariser la croissance des plantes d'un 5-(1,2,4-triazole-1-ylméthyle)-isoxazoline de formule générale I, ses sels et complexes métalliques acceptables sur les plantes, leur biotope ou sur la semence des plantes.

7. Procédé de préparation de 2-(1,2,4-triazole-1-yl-méthyl)-alcène de la formule générale III'

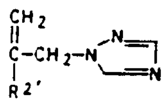

III'

dans laquelle le substituant $R^{2'}$ a les significations suivantes:
un groupe alcoxy en C1-C4-alkyle en C1-C4, un groupe alkyle en C1-C6 partiellement ou totalement halogéné, un groupe alkyle en C1-C6 qui peut porter un reste cycloalkyle en C3-C8 phényle ou naphtyle, un groupe cycloalkyle en C3-C8, phényle ou naphtyle ou un groupe hétéroaryle en C3-C6 qui contient 3 atomes d'azote dont au maximum 2 doivent être voisine, ou jusqu'à deux des hétéroatomes suivants : oxygène, soufre et/ou azote, 2 atomes d'oxygène et/ou soufre ne devant chaque fois pas être voisines, et les parties phényle, naphtyle et hétéroaryle des groupes indiqués pouvant encore porter chacun un reste phényle ou phénoxy ayant jusqu'à 3 substituants halogène ou jusqu'à 3 des restes suivants : halogène, cyano, nitro, alkyle en C1-C4 ou alcoxy en C1-C4 partiellement ou totalement halogénés - excepté méthyle, caractérisé par le fait que l'on fait réagir du méthylène-phosphoniumylite IV

$$(R)_3P=CH_2 \qquad IV$$

où R représente un reste aromatique ou cycloalyphatique en C6 avec le 1,2,4-triazol-1-ylméthylcétone V

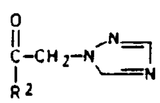

V

8. Procédé de préparation de 2-(1,2,4-triazole-1-yl-méthyl)-alcène de la formule générale III'

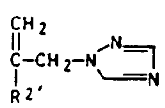

III'

dans laquelle le substituant $R^{2'}$ a les significations suivantes:
un groupe alcoxy en C1-C4-alkyle en C1-C4, un groupe alkyle en C1-C6 partiellement ou totalement halogéné, un groupe alkyle en C1-C6 qui peut porter un reste cycloalkyle en C3-C8 phényle ou naphtyle, un groupe cycloalkyle en C3-C8, phényle ou naphtyle ou un groupe hétéroaryle en C3-C6 qui contient 3 atomes d'azote dont au maximum 2 doivent être voisine, ou jusqu'à deux des hétéroatomes suivants : oxygène, soufre et/ou azote, 2 atomes d'oxygène et/ou soufre ne devant chaque fois pas être voisines, et les parties phényle, naphtyle et hétéroaryle des groupes indiqués pouvant encore porter chacun un reste phényle ou phénoxy ayant jusqu'à 3 substituants halogène ou jusqu'à 3 des restes suivants : halogène, cyano, nitro, alkyle en C1-C4 ou alcoxy en C1-C4 partiellement ou totalement halogénés - excepté méthyle, caractérisé par le fait que l'on fait réagir halogénométhyl-alcène VI'

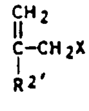

VI'

où H représente hydrogène avec 1,2,4-triazole, en présence d'une base.